# FASCICULE DE BREVET EUROPEEN

(11) **EP 1 797 847 B1**
(45) Date de publication et mention de la délivrance du brevet: **17.06.2009**
(21) Numéro de dépôt: 06125620.2
(22) Date de dépôt: 07.12.2006
(51) Int. Cl.: A61F 7/00

(54) **Procédé de traitement cosmétique ou dermatologique et dispositifs pour la mise en oeuvre d'un tel procédé**
Kosmetische oder dermatologische Behandlungsmethoden und Geräte
Cosmetic or dermatologic treatment process and devices therefor

(30) Priorité: 09.12.2005 FR 0553823
(43) Date de publication de la demande: 20.06.2007
(73) Titulaire: L'Oréal, 75008 Paris (FR)
(72) Inventeur: Gueret, Jean-Louis H., 75016, Paris (FR)
(74) Mandataire: Leray, Noelle

(56) Documents cités:
- US-A- 2 472 385
- US-A- 3 752 155
- US-A- 4 745 909
- US-A1- 2003 100 936

## Description

La présente invention concerne le traitement cosmétique ou dermatologique de la peau avec apport de froid, voire le traitement des autres matières kératiniques telles que les cheveux, par exemple.

Il existe des masques pouvant être placés au réfrigérateur afin de permettre une application de froid sur le visage. De tels masques sont commercialisés sous la dénomination COLDHOT^{®} par la société 3M notamment.

La demande WO 2004/071362 divulgue un masque pour les paupières comportant un polymère absorbant, qui est refroidi avant l'application.

La demande WO 03/055425 décrit un article rafraîchissant comportant des particules de polymère capables d'absorber l'eau et permettant à celles-ci de s'évaporer. Un tel article n'est pas prévu pour appliquer une composition cosmétique ni masser la peau.

Le brevet US 4 745 909 décrit un dispositif destiné à apporter du froid qui comporte un matériau tel que de l'eau contenu dans une enveloppe métallique d'épaisseur constante. Un manchon isolant est emmanché sur une partie de l'enveloppe métallique pour constituer la préhension du dispositif.

Le brevet US 5 127 395 décrit un dispositif destiné à apporter du froid qui comporte une sphère de polyéthylène qui contient un mélange capable de conserver une température de - 20°C.

Le brevet US 4 537 194 décrit un moule pour former des glaçons avec une partie de préhension en matière plastique.

La demande de brevet US 2003/0100936 décrit un rouleau qui peut être empli d'un liquide capable d'emmagasiner la chaleur.

Ces dispositifs ne sont pas associés à un récipient contenant un produit cosmétique à appliquer.

Il a encore été proposé un dispositif de conditionnement capable de refroidir une crème à une température d'environ 2 °C par mélange de deux composants réagissant ensemble. Un tel dispositif de conditionnement est relativement complexe et coûteux, étant de plus réservé à un usage unique.

Il est connu de placer un dispositif d'application contenant une composition de massage et une bille applicatrice au réfrigérateur avant l'utilisation. La bille applicatrice n'est pas utilisable pour masser la peau sans distribuer également la composition de massage.

Le brevet US 4 537 194 divulgue un applicateur agencé pour appliquer une composition à l'état congelé afin de traiter une blessure. L'applicateur comporte un récipient qui peut servir d'organe de préhension une fois la composition congelée.

La possibilité d'introduire une composition cosmétique au réfrigérateur ou au congélateur avant l'application n'est pas sans poser des problèmes de formulation.

En effet, certaines compositions cosmétiques sont susceptibles de se figer et/ou de s'altérer lorsque refroidies par exemple à une température inférieure ou égale à 0 °C.

Par ailleurs, lorsqu'une faible quantité de composition est utilisée localement, l'effet de froid est de courte durée à cause du réchauffement de la composition au contact de la peau.

Enfin, certains dispositifs de distribution sont prévus pour fonctionner avec des compositions présentant des rhéologies particulières et peuvent ne plus fonctionner correctement lorsque la viscosité de la composition est modifiée suite à un changement de température.

Il existe donc un besoin pour pouvoir bénéficier d'un effet de froid durable lors de l'application d'une composition cosmétique ou dermatologique sans être confronté aux inconvénients des procédés et dispositifs connus.

L'invention vise à proposer un procédé de traitement, notamment cosmétique, permettant l'utilisation de froid, qui soit compatible avec une large diversité de compositions cosmétiques.

L'invention a ainsi trait, selon l'un de ses aspects, à un procédé de traitement d'au moins une région du corps humain, comportant les étapes suivantes :
a) refroidir à une basse température inférieure ou égale à 15 °C un applicateur, puis
b) charger l'applicateur ainsi refroidi avec une composition cosmétique ou dermatologique à une température plus proche de la température ambiante que celle de l'applicateur et ayant une différence d'au moins 5 °C avec cette dernière, et
   appliquer la composition en utilisant l'applicateur, ou
c) avant ou après l'étape a), appliquer sur la région à traiter au moins une composition cosmétique ou dermatologique et après l'étape a) amener l'applicateur ainsi refroidi au contact de la région à traiter.

La composition est par exemple appliquée sur la peau juste avant d'amener l'applicateur à son contact. La composition peut encore avoir été appliquée plus d'une heure avant, voire la veille par exemple, selon le traitement à effectuer.

Le procédé peut comporter seulement l'étape b) ou seulement l'étape c).

L'invention peut permettre de traiter une région du corps humain avec apport de froid au moyen d'une composition cosmétique pouvant être quelconque, puisque n'étant refroidie le cas échéant qu'au moment de l'utilisation.

Lorsque la composition est prélevée dans un récipient, un risque de dénaturation, sous l'effet du froid, de la composition non utilisée au sein du récipient la contenant peut ainsi être évité.

L'applicateur peut être utilisé comme outil de massage ou pour effectuer une cryopuncture, entre autres.

L'applicateur peut être utilisé dans un institut de beauté, sur un point de vente, chez un dermatologue ou médecin ou à domicile, par exemple au réveil ou au coucher.

La durée de contact ininterrompu de l'applicateur sur la peau ou les cheveux est par exemple comprise entre 0,5 s et 30 mn.

La durée d'une séance de traitement est par exemple comprise entre 15 s et 1 h, voire plus.

L'apport de froid peut renforcer l'action de la composition cosmétique ou dermatologique, par exemple en préparant l'épiderme à l'action de la composition, en activant la circulation et/ou en favorisant la pénétration des actifs. L'applicateur peut apporter un effet relaxant. L'action exercée peut viser à lisser l'épiderme, le tonifier, le retendre, dégonfler les poches sous les yeux et/ou diminuer les rides, entre autres.

L'applicateur peut être utilisé pour exercer, le cas échéant, une action de massage sans craindre de distribuer un excès de composition sur la région traitée, puisque l'applicateur peut être amené au contact de la région traitée sans que son utilisation ne s'accompagne nécessairement de la distribution de composition sur la région traitée.

La basse température peut être inférieure ou égale à 8 °C, voire inférieure ou égale à 0 °C. L'applicateur est par exemple refroidi initialement à une température comprise entre -18 °C et 0 °C, par exemple -6 °C à -4 °C environ.

L'applicateur peut comporter une cavité, qui peut contenir au moins un composé pouvant changer d'état lorsque refroidi à la basse température et l'applicateur peut être refroidi à une température suffisamment basse pour provoquer ce changement d'état. Ce composé peut être un liquide qui se congèle en étant refroidi. Ce liquide peut comporter de l'eau. Le changement d'état permet d'emmagasiner du froid grâce à la chaleur latente de fusion de la glace. Le liquide peut comporter un additif abaissant son point de congélation, par exemple du chlorure de sodium ou un alcool, par exemple du glycol.

La surface interne de la paroi délimitant la cavité peut être recouverte d'un vernis.

La région à traiter peut être la peau du visage ou du reste du corps, y compris les muqueuses, ou les cheveux. L'applicateur peut permettre éventuellement, lorsque amené au contact des cheveux, d'éliminer les charges électrostratiques, notamment quand la surface d'application est définie par un matériau conducteur de l'électricité, par exemple métallique.

Dans un exemple de mise en oeuvre de l'invention, le procédé comporte l'application sur la région à traiter d'un substrat portant la composition, l'applicateur étant amené au contact du substrat ainsi appliqué. Ce substrat peut comporter un tissé, un non-tissé ou une mousse.

La composition cosmétique peut être prélevée dans un récipient pour être appliquée sur la région à traiter. La composition peut être prélevée au moyen de l'applicateur ou autrement, par exemple avec un doigt ou une spatule ou un dispositif de distribution tel qu'une pompe. Le récipient peut comporter un embout de distribution.

L'applicateur peut être refroidi en étant introduit dans un réfrigérateur ou congélateur ou en étant exposé à une détente d'un gaz comprimé ou liquéfié ou à une réaction endothermique.

L'applicateur peut être encore utilisé, le cas échéant, à température ambiante, si l'utilisateur le souhaite, voire à chaud.

La composition peut être contenue dans un récipient fermé lorsque l'applicateur est utilisé.

L'invention a encore pour objet, selon un autre de ses aspects, un ensemble comportant :
- éventuellement, une composition cosmétique ou dermatologique contenue dans un récipient ou substrat,
- un applicateur pouvant être séparé du récipient ou substrat et ayant une surface d'application pour traiter une région de peau,
l'applicateur comportant au moins un matériau ayant un comportement thermique tel que, lorsque refroidi à une basse température ne causant pas de lésion thermique à la peau lors du contact de la surface d'application avec la peau pendant 15 s, la surface d'application puisse conserver, après cette durée de 15 s au moins de contact avec la peau, une température inférieure à 15 °C.

L'ensemble est limité à un applicateur en l'absence de composition.

La température de la surface d'application peut être mesurée en utilisant un thermomètre sans contact.

L'invention a encore pour objet, selon un autre de ses aspects, un ensemble comportant :
- éventuellement, une composition cosmétique ou dermatologique contenue dans un récipient ou substrat,
- un applicateur à utiliser lors du traitement avec la composition cosmétique, l'applicateur ayant une surface d'application définie au moins partiellement par un matériau présentant une inertie thermique d'au moins 1 000 Jm⁻² K⁻¹ s^{-1/2}, mieux d'au moins 5 000 Jm⁻²K⁻¹ s^{-1/2}, encore mieux d'au moins 10 000 Jm⁻²K⁻¹s^{-1/2}, préférentiellement d'au moins 20 000 Jm⁻² K⁻¹ s^{-1/2}.

L'inertie thermique caractérise l'aptitude de la surface d'application à conserver la basse température lorsqu'exposée périodiquement à un apport de chaleur lors du contact avec la peau.

L'inertie thermique est définie par la formule (k.ρ.C)^{1/2}, où k est la conductivité thermique, p la densité volumique et C la capacité calorifique massique.

On a par exemple pour l'aluminium (en unités SI), ρ = 2702, k = 238 et C = 900, soit une inertie thermique de 24000 J m⁻² K⁻¹ s^{-1/2} environ.

Une inertie thermique relativement élevée permet à la surface d'application de conserver une température relativement faible malgré des contacts répétés avec la peau.

L'invention a encore pour objet, selon un autre de ses aspects, un ensemble comportant :
- éventuellement, une composition cosmétique ou dermatologique contenue dans un récipient ou substrat,
- un applicateur présentant une surface d'application définie au moins partiellement par un matériau présentant une conductivité thermique supérieure ou égale à 1 Wm⁻¹ K⁻¹, mieux à 40 Wm⁻¹ K⁻¹, encore mieux à 180 Wm⁻¹ K⁻¹.

Une conductivité thermique élevée favorise le transfert de froid entre l'applicateur et la région à traiter et permet de bénéficier d'un renouvellement de froid rapide, tant que les frigories emmagasinées par l'applicateur le permettent.

La caractéristique de conductivité thermique est avantageusement combinée à celle relative à l'inertie thermique au sein du même applicateur.

La composition et l'applicateur peuvent être contenus initialement dans un même emballage.

Le récipient et l'applicateur peuvent être contenus initialement dans un même emballage.

L'applicateur et le récipient peuvent, le cas échéant, être contenus dans un dispositif de conditionnement commun, par exemple un coffret, un emballage cartonné, un blister ou un film ou sachet de pelliculage.

L'invention a encore pour objet, selon un autre de ses aspects, un ensemble comportant :
- éventuellement, une composition cosmétique ou dermatologique contenue dans un récipient ou substrat,
- un applicateur ayant une surface d'application et au moins une cavité contenant au moins un composé, par exemple liquide, pouvant éventuellement changer d'état lorsque refroidi de la température ambiante à une température non inférieure à -18 °C, par exemple à une température allant de -18 °C à 0 °C. Ce composé est par exemple un liquide, notamment un liquide contenant de l'eau.

L'invention a encore pour objet, selon un autre de ses aspects, un applicateur comportant :
- une surface d'application définie au moins partiellement par un matériau présentant une conductivité thermique supérieure ou égale à 1 Wm⁻¹ K⁻¹, mieux 40 Wm⁻¹ K⁻¹, encore mieux 180 Wm⁻¹ K⁻¹,
- une surface de préhension définie au moins partiellement par un matériau présentant une conductivité thermique inférieure ou égale à 1 Wm⁻¹ K⁻¹, mieux à 0,5 Wm⁻¹ K⁻¹, encore mieux 0,1 Wm⁻¹ K⁻¹,
- une cavité à l'intérieur de l'applicateur, contenant un liquide, la cavité étant délimitée en partie par le matériau présentant une conductivité thermique supérieure ou égale à 1 Wm⁻¹ K⁻¹ et en partie par le matériau présentant une conductivité thermique inférieure ou égale à 1 Wm⁻¹ K⁻¹.

L'invention a encore pour objet, selon un autre de ses aspects, un applicateur comportant :
- une surface d'application définie au moins partiellement par un matériau présentant une conductivité thermique supérieure ou égale à 1 Wm⁻¹ K⁻¹, mieux 40 Wm⁻¹ K⁻¹, encore mieux 180 Wm⁻¹ K⁻¹,
- une surface de préhension définie au moins partiellement par un matériau présentant une conductivité thermique inférieure ou égale à 1 Wm⁻¹ K⁻¹, mieux à 0,5 Wm⁻¹ K⁻¹, encore mieux 0,1 Wm⁻¹ K⁻¹,
- une cavité à l'intérieur de l'applicateur, contenant un liquide, la cavité étant délimitée par une paroi d'épaisseur non constante.

L'applicateur peut comporter un matériau métallique définissant au moins partiellement la surface d'application destinée à venir au contact de la région à traiter. Ce matériau peut comporter de l'aluminium, éventuellement un alliage tel que le Zamak^{®}. D'autres matériaux sont utilisables, par exemple l'acier inoxydable, le cuivre et leurs alliages.

L'applicateur peut également comporter des matériaux non métalliques mais denses, par exemple de densité supérieure ou égale à 1,1 g/cm³, mieux à 1,5 g/cm³, encore mieux à 2,5 g/cm³, par exemple du sable, du verre ou de la kimberlite.

La surface d'application peut être définie au moins partiellement par un matériau présentant une densité d'au moins 1,5 g/cm³.

La surface d'application peut être définie au moins partiellement par un matériau non métallique, le cas échéant, par exemple du verre.

Comme mentionné ci-dessus, l'applicateur peut comporter un matériau définissant au moins partiellement une surface d'application destinée à venir au contact de la région à traiter et présentant une conductivité thermique supérieure ou égale à 1 Wm⁻¹ K⁻¹, mieux 40 Wm⁻¹ K⁻¹, encore mieux supérieure ou égale à 180 Wm⁻¹ K⁻¹. Ce matériau peut être en contact avec le liquide contenu dans la cavité précitée, le cas échéant.

L'applicateur peut comporter un matériau de capacité calorifique massique supérieure ou égale à 500 J kg⁻¹ K⁻¹, mieux à 1 000 J kg⁻¹ K⁻¹, encore mieux à 2000 J kg⁻¹ K⁻¹.

La capacité thermique de l'applicateur est par exemple suffisante pour qu'à température ambiante de 20 °C la surface d'application conserve pendant au moins 10 minutes une température inférieure ou égale à 15 °C lorsque refroidie initialement à -6 °C.

L'applicateur peut présenter une masse supérieure ou égale à 15 g, en incluant le liquide éventuel.

L'applicateur peut comporter au moins 0,2 cm³ de liquide dans la cavité, par exemple entre 1 cm³ et 80 cm³, notamment entre 5 cm³ et 70 cm³, par exemple entre 5 cm³ et 15 cm³, voire entre 5 cm³ et 10 cm³.

L'applicateur peut comporter une surface de préhension définie au moins partiellement par un matériau présentant une conductivité thermique inférieure ou égale à 1 Wm⁻¹K⁻¹, mieux à 0,5 Wm⁻¹K⁻¹ , encore mieux à 0,1 Wm⁻¹K⁻¹, par exemple une matière non métallique ou non minérale telle que le plastique ou le bois. La surface de préhension peut également être définie par un matériau alvéolaire, par exemple une mousse.

La cavité précitée peut être délimitée en partie par un matériau présentant une conductivité thermique supérieure ou égale à 1 Wm⁻¹ K⁻¹ et en partie par un matériau présentant une conductivité thermique inférieure ou égale à 1 Wm⁻¹ K⁻¹. La cavité peut être délimitée en partie par un matériau métallique et en partie par un matériau plastique.

La plus grande partie de la cavité peut être délimitée par un matériau présentant une conductivité thermique inférieure ou égale à 1 Wm⁻¹ K⁻¹.

L'applicateur peut comporter au moins une partie qui est moulée, par exemple par injection ou soufflage, ou qui est usinée.

La cavité peut être réalisée par moulage et/ou par usinage. L'applicateur peut comporter plus d'une cavité contenant le liquide pouvant changer d'état.

L'applicateur peut comporter un matériau métallique et une matière plastique, un verre et un matériau métallique, un verre et une matière plastique, par exemple.

La surface d'application peut être douce et polie ou en variante, comporter des aspérités ou reliefs tels que des picots. Les reliefs peuvent être choisis en fonction par exemple du froid que l'on souhaite apporter lors du contact de l'applicateur avec la région à traiter. Les reliefs peuvent permettre de diminuer l'étendue du contact avec la peau.

La surface d'application peut être définie par un matériau dur ou non. Le cas échéant, la surface d'application peut être définie au moins partiellement par une paroi au moins partiellement recouverte d'une membrane élastomère, d'une mousse, d'un flocage, d'un film de matière plastique, d'une éponge, d'un feutre, d'un tissé ou non-tissé. Cette paroi ainsi recouverte est par exemple constituée au moins partiellement par un matériau métallique.

La surface d'application peut avoir au moins une zone d'étendue comprise entre 3 et 10 cm² pouvant venir entièrement au contact avec la peau et la capacité thermique de l'applicateur peut être supérieure ou égale à 250 J K⁻¹ à une température de 15 °C, ou au moins un zone d'étendue comprise entre 0,1 et 3 cm² pouvant venir entièrement au contact avec la peau et la capacité thermique de l'applicateur peut être supérieure ou égale à 50 J K⁻¹ à une température de 15 °C.

L'applicateur peut être agencé pour se fixer de manière amovible sur le récipient contenant la composition à appliquer. L'applicateur peut être agencé pour se fixer de manière amovible sur un organe de fermeture du récipient. Le récipient comporte par exemple un couvercle présentant un logement dans lequel peut être inséré l'applicateur en l'absence d'utilisation. L'applicateur peut éventuellement servir d'organe de fermeture à un récipient contenant la composition.

L'applicateur peut encore être agencé pour se fixer de manière amovible sur un dispositif de distribution permettant de prélever la composition.

Le récipient peut encore comporter une extension ayant un logement pouvant recevoir l'applicateur.

L'applicateur peut être agencé pour, en position de stockage, pouvoir reposer sur une surface de manière à ce que le liquide soit en contact avec la paroi délimitant la surface d'application.

L'applicateur peut comporter un organe d'application rotatif agencé pour venir au contact de la région à traiter, voire une pluralité d'organes d'application rotatifs, lesquels peuvent plisser et déplacer la peau lors de leur passage, par exemple.

L'applicateur peut être au moins partiellement magnétique.

L'applicateur peut comporter une lèvre flexible, par exemple agencée à la manière d'une ventouse.

L'applicateur peut comporter un passage permettant à la composition d'être distribuée quand l'applicateur est monté sur le récipient. La composition peut venir au contact de ce passage ou ce dernier peut recevoir un embout de distribution dans lequel circule la composition.

L'applicateur peut comporter un vibreur et/ou au moins une électrode reliée à une source électrique, par exemple deux électrodes entre lesquelles existe une différence de potentiel. Le récipient peut présenter un espace intérieur contenant la composition, de volume variable.

L'applicateur peut présenter une surface d'application définie au moins partiellement par une pièce amovible. Cette dernière comporte par exemple un matériau absorbant et/ou alvéolaire et/ou des fibres, ou poils, et peut par exemple être détachée de l'applicateur pour être lavée. La pièce amovible peut par exemple améliorer l'étalement de la composition sur la région traitée au moyen de l'applicateur.

L'applicateur peut présenter entre la cavité précitée et la surface d'application une plus petite épaisseur (eₘᵢₙ) inférieure ou égale à 50 mm, mieux inférieure ou égale à 10 mm, encore mieux inférieure ou égale à 1 mm, par exemple comprise entre 0,1 mm et 1 mm, par exemple allant de 0,2 mm à 0,8 mm, afin de favoriser le transfert thermique entre la surface d'application et le liquide contenu dans la cavité.

L'applicateur peut comporter au moins un indicateur de température, par exemple par changement de couleur. L'indicateur de température peut changer de couleur pour signaler à l'utilisateur que la surface d'application est à une température supérieure ou inférieure à un seuil prédéfini, par exemple.

L'applicateur peut comporter un organe de prélèvement de la composition contenue dans le récipient. Cet organe de prélèvement comporte par exemple une mousse, un fritté, un feutre, un tissé, un non-tissé, un flocage ou des poils. L'organe de prélèvement peut se situer à une extrémité de l'applicateur opposée à la surface d'application.

L'ensemble peut être agencé pour, lorsque refroidi à -8 °C et appliqué sur la peau, conserver pendant au moins 30 s, mieux 1 mn, encore mieux 15 mn ou 30 mn, une température inférieure ou égale à 15 °C.

L'applicateur peut ne pas comporter de composés réagissant ensemble selon une réaction endothermique.

L'applicateur peut être dépourvu de source électrique ou de moyen de raccordement à une source électrique.

L'applicateur peut comporter une première partie assemblée avec au moins une deuxième partie, par exemple par montage à force, encliquetage, vissage, soudage, collage, surmoulage ou sertissage.

La première partie peut définir la surface d'application et peut comporter un matériau métallique, par exemple. La deuxième partie peut définir la surface de préhension et peut comporter un matériau non métallique. La première ou la deuxième partie peut comporter une ouverture de remplissage de la cavité contenant le liquide, le cas échéant. En variante, la cavité peut être remplie avant assemblage des première et deuxième parties.

Le récipient peut comporter un premier compartiment contenant la composition et un deuxième compartiment pour recevoir l'applicateur et un organe de fermeture permettant de fermer à la fois les premier et deuxième compartiments.

Le récipient peut encore comporter un logement central pour recevoir au moins partiellement l'applicateur.

L'ensemble de conditionnement et d'application peut comporter un premier applicateur destiné à être refroidi et un deuxième applicateur destiné à être réchauffé. Le premier applicateur peut contenir un liquide et comporter un matériau métallique et le deuxième applicateur être dépourvu de métal, afin de pouvoir être introduit dans un four à micro-ondes.

L'invention a encore pour objet, selon un autre de ses aspects, un ensemble comportant :
- éventuellement une composition cosmétique ou dermatologique,
- un applicateur agencé pour, lorsque refroidi à -8 °C et appliqué sur la peau, conserver pendant au moins 30 s, mieux 1 mn, encore mieux 15 mn ou 30 mn, une température inférieure ou égale à 15 °C.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente en élévation un dispositif réalisé conformément à un exemple de mise en oeuvre de l'invention,
- la figure 2 est une coupe longitudinale, schématique, du dispositif de la figure 1,
- la figure 3 représente isolément l'applicateur des figures 1 et 2,
- les figures 4, 5 et 6 sont des vues analogues à la figure 3 de variantes de réalisation de l'applicateur,
- la figure 6 est une vue analogue à la figure 1 d'une variante de réalisation du dispositif,
- la figure 7 représente isolément, en élévation, l'applicateur de la figure 6,
- la figure 8 est une vue en perspective d'une variante de réalisation du dispositif,
- la figure 9 est une coupe longitudinale du dispositif de la figure 8,
- les figures 10 à 14 sont des coupes longitudinales, schématiques et partielles, de dispositifs selon des variantes de mise en oeuvre de l'invention,
- les figures 15 et 16 représentent de manière schématique, en perspective, des exemples de kits pour la mise en oeuvre de l'invention,
- la figure 17 illustre l'utilisation du kit de la figure 16,
- la figure 18 représente de manière schématique un autre exemple de kit pour mettre en oeuvre l'invention,
- la figure 19 représente l'applicateur de la figure 18 au moment de l'utilisation,
- les figures 20 à 25, 27 à 29 et 60 sont des coupes longitudinales, schématiques, de variantes de réalisation de l'applicateur,
- la figure 26 représente des variantes de réalisation de l'applicateur,
- la figure 30 représente de manière schématique, en perspective, un autre exemple de réalisation de l'applicateur,
- la figure 31 est une coupe longitudinale partielle de l'applicateur de la figure 30,
- les figures 32 à 35 sont des vues en élévation de variantes de réalisation de l'applicateur,
- les figures 36 à 53 sont des coupes longitudinales, partielles et schématiques, de variantes de réalisation de l'applicateur,
- la figure 54 représente, en perspective, un autre exemple de dispositif de conditionnement et d'application,
- la figure 55 est une coupe schématique et partielle de l'applicateur,
- la figure 56 est une vue analogue à la figure 54 d'une variante de réalisation, et
- les figures 57 à 66 illustrent des variantes de mise en oeuvre de l'invention.

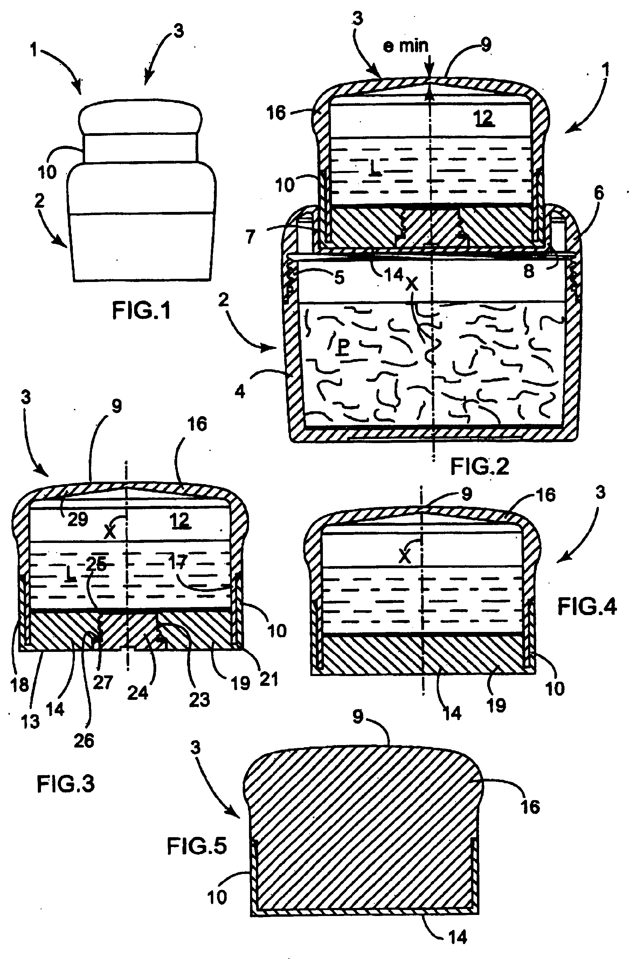

Le dispositif 1 de conditionnement et d'application représenté aux figures 1 et 2 comporte un récipient 2 contenant une composition P à appliquer sur le corps humain et un applicateur 3, d'axe X, pouvant être utilisé lors de l'application de la composition P.

La composition P est par exemple destinée à exercer au moins une action anti-ride, amincissante, hydratante, colorante, anti-acnéique, anti-séboréique, dépigmentante, stimulante, régénérante, apaisante et/ou à dissimuler des défauts cutanés.

La composition P est par exemple une composition cosmétique telle que définie dans la Directive 93/35/CEE du 14 juin 1993 modifiant la Directive 76/768/CEE.

La composition P est par exemple telle qu'elle ne supporte pas un stockage prolongé dans le récipient 2 à une température inférieure ou égale à 0 °C.

Dans l'exemple considéré, le récipient 2 se présente sous la forme d'un pot comportant un corps 4 pourvu supérieurement d'un col fileté 5 et d'un couvercle 6 pouvant se fixer de façon amovible sur le col 5, par exemple par vissage.

Le couvercle 6 et le col 5 peuvent comporter des moyens d'étanchéité permettant d'obtenir une fermeture étanche du récipient 2.

Le couvercle 6 comporte une paroi supérieure 8 définissant un logement 7 ouvert vers le haut, dans lequel peut s'engager l'applicateur 3 en l'absence d'utilisation.

L'applicateur 3 est par exemple retenu par friction dans le logement 7.

L'applicateur 3 présente une surface d'application 9 destinée à venir au contact de la région à traiter, par exemple la peau, et une surface de préhension 10 pouvant être saisie par l'utilisateur pour le manipuler.

La surface d'application 9 est par exemple située du côté opposé au récipient 2 lorsque l'applicateur 3 est reçu dans le logement 7.

La surface de préhension 10 est par exemple une surface latérale s'étendant à partir d'une face inférieure 13 de l'applicateur, par exemple sur plus du tiers de la hauteur totale de l'applicateur, par exemple sur sensiblement la moitié de sa hauteur totale.

Dans l'exemple considéré, l'applicateur 3 comporte une cavité intérieure 12 qui peut contenir un liquide L destiné à emmagasiner du froid.

Le liquide L est avantageusement choisi de façon à changer d'état au cours du refroidissement de l'applicateur 3.

Le liquide L peut par exemple comporter de l'eau et passer d'un état liquide à un état congelé lors du refroidissement de l'applicateur 3 à une température inférieure ou égale à 0 °C.

La quantité de liquide L va par exemple de 0,2 à 10 cm³, en fonction de la chaleur ou du froid que l'on cherche à emmagasiner.

Le liquide peut comporter un composé abaissant son point de congélation.

Le liquide L peut changer d'état par exemple entre -12 °C et 0 °C.

Le volume de la cavité 12 peut être supérieur à celui du liquide L, de façon à permettre une expansion de celui-ci lors de la congélation.

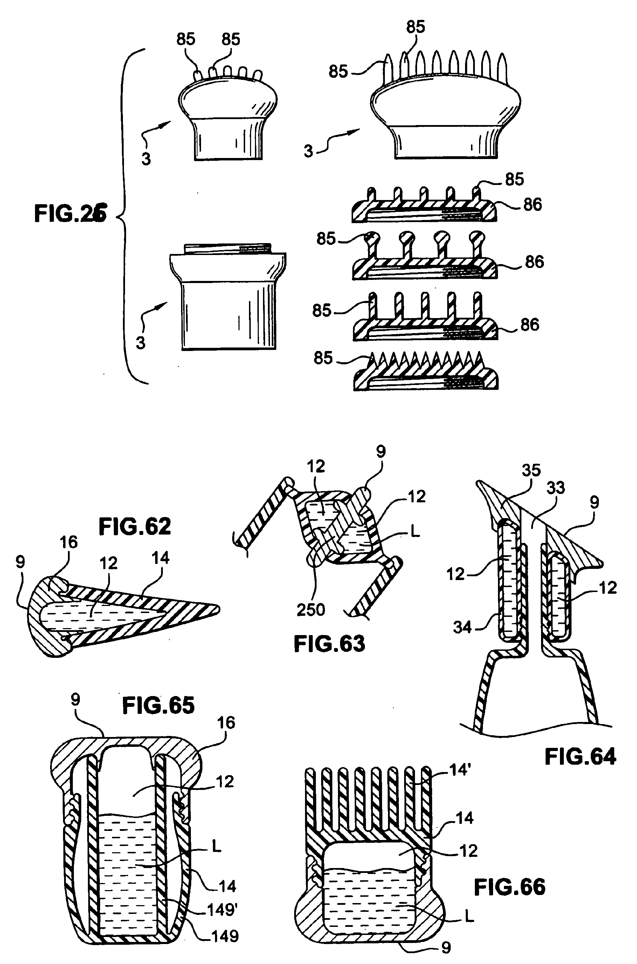

L'applicateur est avantageusement configuré pour pouvoir reposer sur une surface de manière à ce que le liquide soit en contact avec la paroi délimitant la surface d'application. Par exemple, la surface d'application 9 est sensiblement plane de manière à pouvoir reposer sur une surface plane comme on l'a illustré à la figure 66. L'applicateur peut alors être placé dans cette position « tête en bas » au réfrigérateur puis, entre deux utilisations, de façon que le liquide reste en contact avec la surface d'application 9.

Dans une variante non illustrée, la cavité 12 est remplie d'une poudre, par exemple du sable.

Dans l'exemple considéré, la cavité intérieure 12 est formée entre une première partie 16 qui définit la surface d'application 9 et une deuxième partie 14 qui définit la surface de préhension 10, cette deuxième partie 14 étant assemblée avec la première.

La deuxième partie 14 comporte par exemple une jupe extérieure 18 d'axe X qui vient recouvrir une jupe intérieure 17 de la première partie 16 et qui se raccorde à une paroi transversale 19, laquelle forme avec la jupe extérieure 18 une gorge annulaire 21 dans laquelle s'engage la jupe intérieure 17. Cela peut faciliter l'obtention d'un assemblage étanche.

La deuxième partie 14 est avantageusement réalisée dans une matière non métallique, par exemple une matière plastique, afin d'isoler thermiquement la surface de préhension 10 de la cavité 12.

La deuxième partie 14 est par exemple réalisée dans une matière plastique présentant une conductivité thermique inférieure ou égale à 1 Wm⁻¹K⁻¹, mieux inférieure ou égale à 0,5 Wm⁻¹K⁻¹.

La deuxième partie 14 peut comporter une ouverture 23 permettant le remplissage de la cavité 12 avec le liquide L. Cette ouverture 23 est par exemple réalisée à travers la paroi transversale 19 et elle peut être obturée par un bouchon 24.

L'ouverture 23 présente par exemple une portion filetée 25 et un épaulement 26. Le bouchon 24 peut être vissé dans la portion filetée 25 et un joint d'étanchéité 27 peut s'interposer entre le bouchon 24 et l'épaulement 26.

La première partie 16 comporte une paroi supérieure 29 dont l'épaisseur, mesurée selon l'axe X, peut par exemple décroître en rapprochement de l'axe X de façon à favoriser le transfert thermique entre la cavité 12 et la surface d'application 9 dans la région centrale de l'applicateur 3.

La paroi supérieure 29 présente par exemple sur l'axe X une plus petite épaisseur eₘᵢₙ inférieure ou égale à 1 mm, par exemple de l'ordre de 0,2 à 0,5 mm.

La première partie 16 est par exemple réalisée dans un métal bon conducteur de la chaleur et d'inertie thermique relativement élevée, par exemple de l'aluminium ou un alliage d'aluminium tel que le Zamak^{®}.

La cavité 12 peut être fermée de façon étanche autrement qu'au moyen d'un bouchon rapporté.

Dans la variante illustrée à la figure 4, la deuxième partie 14 ne comporte pas l'ouverture 23, les première et deuxième parties étant par exemple assemblées après remplissage de la cavité 12 avec le liquide L et coopérant de façon à fermer de façon étanche la cavité 12.

L'assemblage des première et deuxième parties, dans les exemples des figures 3 et 4 entre autres, peut se faire de diverses façons, par exemple par montage en force, collage, encliquetage, soudure, surmoulage, vissage, ou sertissage.

Dans la variante illustrée à la figure 5, l'applicateur 3 ne comporte pas la cavité 12 remplie du liquide L.

La première partie 16 est alors par exemple pleine et réalisée dans un matériau présentant une capacité thermique et une conductivité thermique suffisantes pour obtenir le résultat recherché. Ce matériau comporte par exemple un matériau métallique, un verre, une pierre dense ou une matière plastique chargée.

La deuxième partie 14 peut être utile pour isoler thermiquement la surface de préhension 10 et peut être réalisée dans une matière plastique ou en bois, par exemple.

La deuxième partie 14 peut aussi comporter une double paroi 149, 149' comme illustré à la figure 65. Cette double paroi contribue à l'isolation thermique de la surface de préhension.

La deuxième partie 14 peut aussi comporter des ailettes 14', comme illustré à la figure 66, qui permettent de réduire la contenance de l'applicateur tout en permettant une bonne préhension.

L'applicateur 3 peut encore, dans une variante non illustrée, être réalisé d'une seule pièce en un seul matériau.

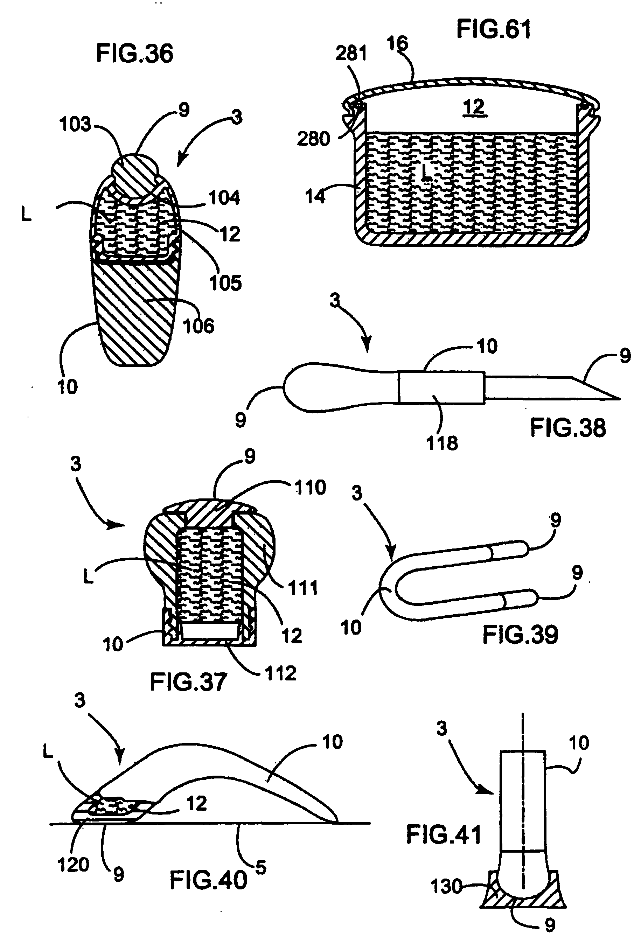

Dans la variante de la figure 61, la première partie 16 est par exemple formée d'une plaque emboutie, par exemple une plaque métallique, notamment d'aluminium.

La deuxième partie 14 est par exemple agencée pour permettre la fixation de la première partie 16 par encliquetage.

La deuxième partie 14 peut comporter une gorge 180 pouvant accueillir un joint d'étanchéité 281 s'appliquant sur la surface intérieure de la première partie 16. Cette dernière peut être fixée sur la deuxième partie 14 après remplissage de la cavité 12 par le liquide L.

L'applicateur 3 peut être réalisé avec une forme adaptée à la région à traiter.

Les applicateurs 3 représentés sur les figures 1 à 5 sont relativement larges, étant par exemple plus larges que hauts, et sont destinés par exemple à traiter une région du corps autre que le visage.

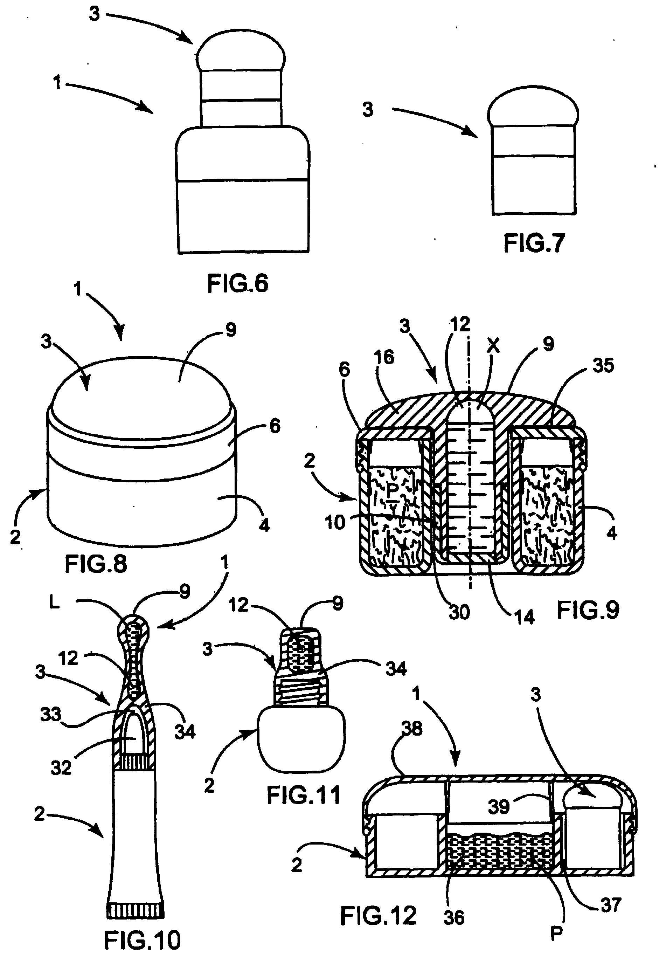

On a représenté à la figure 6 un dispositif de conditionnement et d'application 1 ayant un applicateur 3 plus étroit, davantage adapté au visage. On peut voir sur la figure 7 que cet applicateur 3 est par exemple plus haut que large.

L'applicateur de la figure 4 contient par exemple 50 ml d'eau. La première partie 16 est par exemple formée de 42,9 g d'aluminium et la deuxième partie de 13,9 g de polyacétal (POM).

La capacité thermique de l'applicateur à 15 °C, l'eau étant liquide, vaut sensiblement (50 10⁻³.4185) + (13,9 10⁻³.1460) + (42,9 10⁻³.900) ≃ 268 J K⁻¹ et la chaleur latente de fusion de la glace 50 10⁻³.3,34 10⁵ ≃ 16 700 J.

L'applicateur de la figure 7 contient par exemple 9,5 ml d'eau. La première partie 16 est formée par exemple de 15,1 g d'aluminium et la deuxième partie de 3,6 g de polyacétal (POM).

La capacité thermique de l'applicateur à 15 °C, l'eau étant liquide, vaut sensiblement (9,5 10⁻³.4185) + (3,6 10⁻³.1460) + (15,1 10⁻³.900) ≃ 58,6 J K⁻¹.

On a représenté aux figures 8 et 9 un dispositif selon une variante de réalisation dans laquelle le récipient 2 présente un logement central 30 pour permettre d'y engager au moins partiellement l'applicateur 3.

Le logement 30 est par exemple traversant, comme illustré.

L'applicateur 3 est par exemple agencé pour reposer par une face d'appui 35 contre le dessus du couvercle du récipient 2, l'applicateur 3 présentant par exemple une forme générale de champignon. La face d'appui 35 peut ainsi présenter une forme annulaire.

L'applicateur 3 de la figure 9 peut être réalisé comme illustré avec la cavité 12 et celle-ci s'étend par exemple au moins partiellement dans le logement 30 lorsque l'applicateur 3 est en place sur le récipient 2.

La face d'appui 35 peut être définie par la première partie 16.

Pour utiliser les applicateurs 3 des exemples des figures 1 à 9, l'utilisateur peut les refroidir en les plaçant au congélateur, de façon à congeler le liquide L lorsque celui-ci est présent.

A la sortie du congélateur, la température de la surface d'application est par exemple comprise entre -6 °C et -8 °C.

Le récipient 2 contenant la composition cosmétique P peut être maintenu à une température supérieure à celle de l'applicateur 3, par exemple en étant conservé à température ambiante hors du réfrigérateur.

Ensuite, l'utilisateur peut prélever la composition P dans le récipient 2, par exemple avec le doigt, et l'appliquer sur la région à traiter ou sur l'applicateur 3.

Ensuite, l'applicateur 3 peut être amené au contact de la région à traiter.

L'applicateur 3 peut être utilisé en étant déplacé au contact de la peau, par exemple par des mouvements circulaires ou rectilignes, pour exercer, par exemple, une action de massage et/ou, le cas échéant, étaler la composition P. L'utilisateur peut encore procéder par touches successives, sans mouvement sensible de l'applicateur sur la peau, pour effectuer par exemple une cryopuncture. L'applicateur 3 peut encore effleurer la peau. L'applicateur 3 peut être encore utilisé pour effectuer une sorte de repassage à froid de la peau ou d'un substrat sur la peau.

L'applicateur 3 peut être utilisé pour traiter les poches sous les yeux.

L'applicateur 3 peut être utilisé, le cas échéant, pour prélever la composition P dans le récipient 2.

On a illustré à la figure 10 la possibilité pour le récipient 2 de se présenter sous la forme d'un tube ayant un embout de distribution obturé par un capuchon de fermeture 32.

L'applicateur 3 peut être agencé pour se fixer sur le capuchon de fermeture 32 grâce à un évidement 33 réalisé dans le corps 34 de l'applicateur.

Le maintien de l'applicateur 3 sur le capuchon 32 peut être assuré par exemple par friction ou autrement, par exemple par encliquetage ou vissage.

Le corps 34 peut être réalisé avec la cavité 12 recevant le liquide L, comme illustré.

Pour utiliser l'applicateur 3 de la figure 10, l'utilisateur peut le séparer du récipient 2 et le placer au réfrigérateur par exemple.

Ensuite, l'utilisateur peut distribuer la composition contenue dans le récipient 2 sur l'applicateur 3 ou sur la région à traiter.

L'utilisateur peut se servir du corps 34 comme d'un organe de préhension sans replacer l'applicateur 3 sur celui-ci ou en variante utiliser l'applicateur 3 après l'avoir fixé sur le récipient 2, ce dernier définissant alors la surface de préhension.

Selon une variante illustrée à la figure 64, l'applicateur 3 est par exemple agencé pour se fixer directement sur l'embout de distribution du tube par friction, grâce à un évidement 33, qui est cette fois débouchant de manière à former un passage pour le produit. Le corps 34 de l'applicateur comporte une cavité 12 autour de l'évidement 33 de sorte que, une fois l'applicateur monté sur le tube, la cavité entoure l'embout de distribution. Le corps 34 de l'applicateur est surmonté par une tête 35 métallique qui vient au contact de la peau lors de la distribution du produit.

On a représenté à la figure 11 une variante de réalisation dans laquelle le récipient 2 est un flacon pourvu d'un col et le corps 34 sert de bouchon de fermeture en étant vissé sur le col.

La composition P peut encore être contenue dans un récipient 2 tel que celui illustré à la figure 12, qui comporte un premier compartiment 36 pour recevoir la composition P et un deuxième compartiment 37 pour recevoir l'applicateur 3.

Ces deux compartiments 36 et 37 sont dans l'exemple considéré obturés par un couvercle commun 38, lequel comporte par exemple une jupe d'étanchéité 39 afin de fermer de façon étanche le premier compartiment 36.

Dans l'exemple de la figure 12, le couvercle 38 est agencé pour se visser mais dans des variantes non illustrées, le couvercle 38 se fixe autrement, étant par exemple emboîté ou retenu par une articulation sur le récipient 2.

Le deuxième compartiment 37 peut, le cas échéant, contenir plusieurs applicateurs 3 ayant des formes différentes et/ou destinés à être utilisés différemment, par exemple en étant refroidi pour au moins l'un d'entre eux et réchauffé pour au moins un autre d'entre eux.

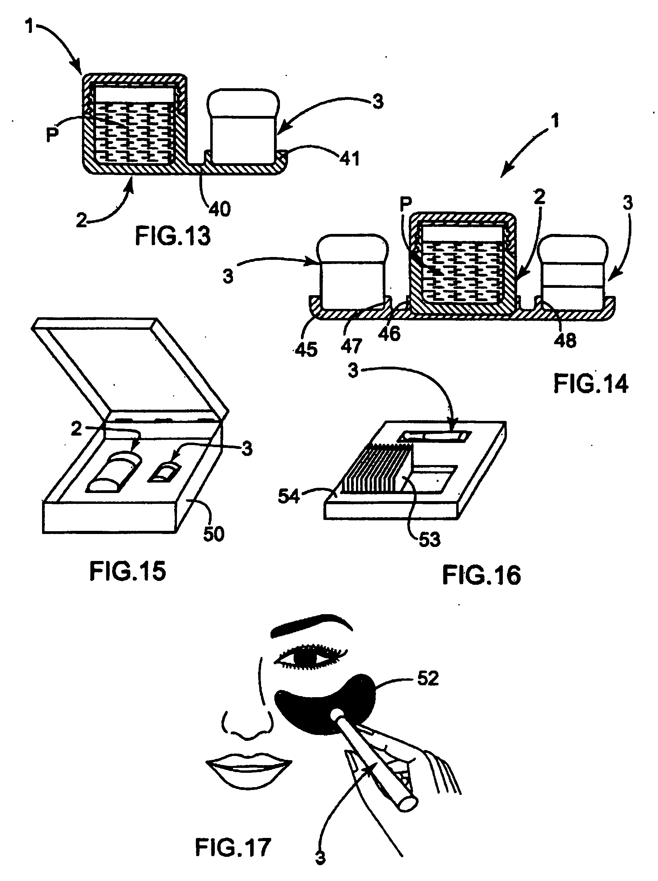

La composition P peut, comme illustré à la figure 13, être contenue dans un récipient 2 comportant une extension 40 agencée pour accueillir l'applicateur 3.

L'extension 40 s'étend par exemple latéralement et peut comporter un logement 41 de forme adaptée à recevoir l'applicateur 3.

La composition P à appliquer peut être proposée à l'utilisateur associée à au moins deux applicateurs 3, l'un étant par exemple destiné à être refroidi et l'autre à être réchauffé.

Ces deux applicateurs 3 peuvent être proposés à l'utilisateur sous la forme d'un kit avec le récipient 2 contenant la composition P.

Le kit comporte, par exemple, un support 45 pouvant accueillir le récipient 2 et les applicateurs 3 en l'absence d'utilisation, comme illustré à la figure 14.

Le support 45 peut comporter par exemple un logement 46 pour recevoir le récipient 2 contenant la composition P et deux logements 47 et 48 pour recevoir chacun un applicateur 3.

Le récipient 2 est réalisé d'une seule pièce avec le support 45 dans une variante non illustrée.

Le récipient 2 peut également être proposé à l'utilisateur avec au moins un applicateur 3 dans un emballage tel que par exemple un coffret 50, comme illustré à la figure 15.

La composition P peut se présenter sous la forme d'une poudre, crème, pâte, gel ou liquide, ou encore imprégner et/ou recouvrir un substrat tel que par exemple un tissé, un non-tissé, une mousse ou un feutre.

Ce substrat 52, sous la forme d'un masque ou d'un patch par exemple, peut être appliqué sur la peau, puis l'applicateur 3 refroidi être amené à son contact, comme illustré à la figure 17.

Le substrat 52 peut être contenu dans un emballage individuel 53 et être proposé à l'utilisateur sous la forme d'un kit en association avec au moins un applicateur 3, l'ensemble étant par exemple contenu dans un emballage 54, comme illustré à la figure 16.

Une pluralité de substrats peut encore être proposée à l'utilisateur, non pas dans des emballages individuels mais dans un emballage commun, dans une variante non illustrée.

Le substrat imprégné de composition peut éventuellement être sensiblement anhydre et peut avoir à être mouillé par un solvant tel que de l'eau par exemple, au moment de l'utilisation.

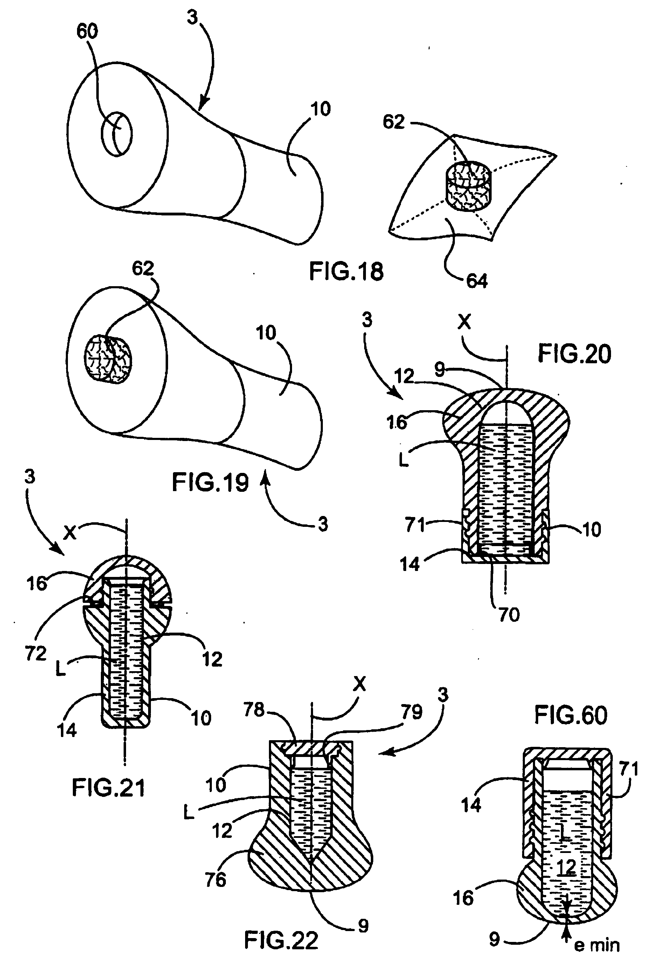

On a représenté à la figure 18 un autre exemple de kit pour la mise en oeuvre de l'invention.

L'applicateur 3 comporte un logement 60 destiné à recevoir un substrat 62 contenant la composition à appliquer, voire la composition elle-même sous la forme d'un bloc de forme adaptée à être reçue dans le logement 60.

Le substrat 62 ou le bloc de composition peut être contenu dans un emballage individuel 64, par exemple.

La quantité de composition contenue dans le substrat 62 ou constituant le bloc précité peut correspondre à un usage unique.

Lors de l'utilisation, l'utilisateur extrait le substrat 62 ou le bloc de composition de l'emballage 64 et l'introduit dans le logement 60 de l'applicateur.

La profondeur du logement 60 est par exemple inférieure à celle du substrat 62 de façon à ce que l'utilisateur puisse appliquer la composition sur la région à traiter.

Le logement 60 est par exemple réalisé dans une partie métallique de l'applicateur 3, afin de favoriser l'échange de chaleur entre le substrat 62 ou le bloc de composition et l'applicateur 3.

La mise en place du substrat 62 ou du bloc de composition peut s'effectuer sur l'applicateur refroidi ou réchauffé.

Après l'application, le substrat 62 peut être retiré du logement 60.

L'applicateur 3 peut être réalisé de multiples autres façons encore.

La cavité 12 qui contient le liquide L peut notamment être fermée de diverses manières.

On a représenté à la figure 20 un exemple de réalisation dans lequel les première 16 et deuxième 14 parties coopèrent par vissage et la deuxième partie 14 comporte une lèvre d'étanchéité 70 qui vient s'appliquer sur une surface radialement intérieure de la première partie afin de fermer de façon étanche la cavité 12.

La deuxième partie 14 peut comporter une jupe filetée 71 qui recouvre partiellement la première partie 16 et qui définit la surface de préhension 10.

Dans la variante de réalisation de la figure 21, les première 16 et deuxième 14 parties sont assemblées avec interposition d'un joint d'étanchéité 72.

La deuxième partie 14 est par exemple filetée du côté de la première partie 16, laquelle peut être vissée sur la deuxième partie 14.

L'applicateur 3 peut encore comporter un corps 76 définissant les surfaces de préhension 10 et d'application 9, comme illustré à la figure 22. Ce corps peut être évidé pour définir la cavité 12 remplie du liquide L.

La cavité 12 peut être fermée par exemple au moyen d'un bouchon 78 qui peut être fixé de diverses manières sur le corps 76, étant par exemple encliqueté sur celui-ci. Le bouchon 78 peut comporter une lèvre annulaire d'étanchéité 79 s'appliquant sur une surface du corps 76 délimitant la cavité 12.

On a représenté à la figure 60 une variante de réalisation assez similaire à celle de la figure 20. Toutefois, la deuxième partie 14 comporte une jupe 71 qui s'étend jusqu'à la portion renflée de la première partie. En outre, l'épaisseur de la première partie 16 entre la cavité 12 et la surface d'application est plus faible.

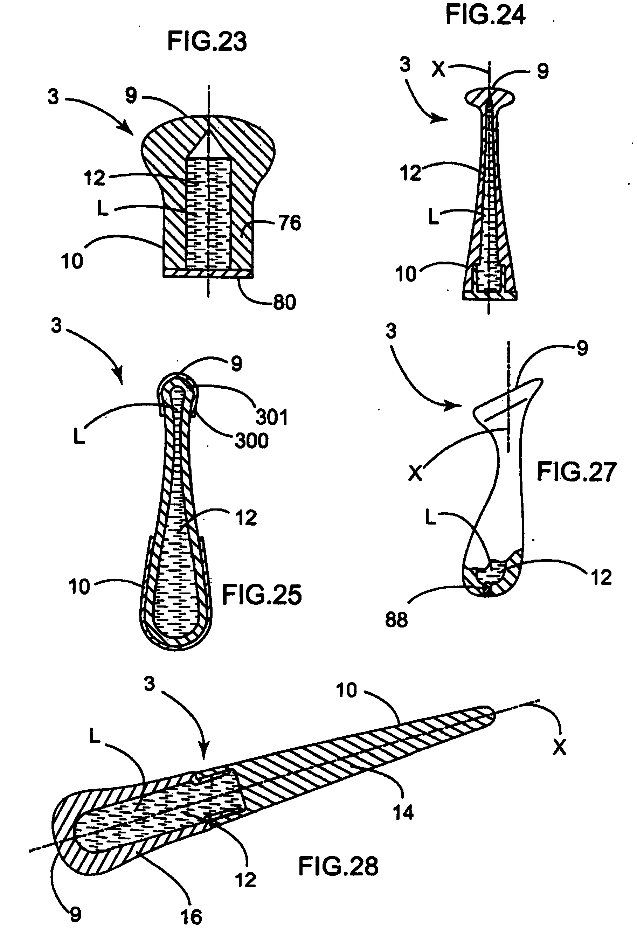

L'applicateur 3 représenté à la figure 23 diffère de celui illustré à la figure 22 par le fait que la cavité 12 est fermée par un fond 80 qui est par exemple soudé sur le corps 76 autour de l'ouverture servant au remplissage de la cavité 12.

Le fond 80 est par exemple une feuille d'un matériau imperméable au liquide L, qui peut être transparent le cas échéant.

L'applicateur 3 peut être réalisé avec des formes extérieures très différentes, par exemple une forme allongée avec une tête élargie, comme illustré à la figure 24.

La cavité 12 peut s'étendre sur une hauteur plus ou moins importante de l'applicateur.

Dans l'exemple de la figure 24, la cavité 12 s'étend sur plus de la moitié de la longueur de l'applicateur 3, en l'espèce pratiquement sur toute sa longueur.

Dans l'exemple de réalisation de la figure 25, l'applicateur 3 présente une tête arrondie.

On a illustré sur cette figure la possibilité pour l'applicateur de comporter une surface d'application 9 définie par un revêtement 300 recouvrant une paroi 301 délimitant la cavité 12. Le revêtement 300 est par exemple une membrane en élastomère, un tissé ou un non-tissé.

La surface d'application 9 de l'applicateur 3 peut comporter des reliefs 85, par exemple des picots comme illustré à la figure 26.

Les reliefs 85 peuvent être réalisés dans un matériau élastomère ou métallique, par exemple.

Les reliefs 85 peuvent modifier le transfert de froid et leur forme peut être choisie en fonction du traitement à effectuer.

Le cas échéant, les reliefs 85 peuvent être réalisés sur une pièce amovible 86, comme illustré, ce qui permet à l'utilisateur de choisir les reliefs 85 en fonction du traitement à effectuer. La fixation de la pièce 86 peut s'effectuer par vissage, par exemple.

L'applicateur 3 peut comporter une surface d'application 9 ayant une portion plane comme illustré à la figure 27, laquelle peut s'étendre obliquement par rapport à l'axe longitudinal X de l'applicateur 3, par exemple.

On peut voir également sur la figure 27 que la cavité 12 peut comporter une ouverture pour son remplissage avec le liquide L, ouverture qui peut être obturée par un bouchon 88 formé par exemple par une goutte de colle.

On a représenté à la figure 28 un applicateur 3 qui comporte une première partie 16 définissant la cavité 12 remplie du liquide L et une deuxième partie 14 se présentant sous la forme d'un manche allongé qui peut ne pas être parcouru par la cavité 12, comme illustré. En variante, la deuxième partie 14 est parcourue par la cavité 12 comme illustré à la figure 62.

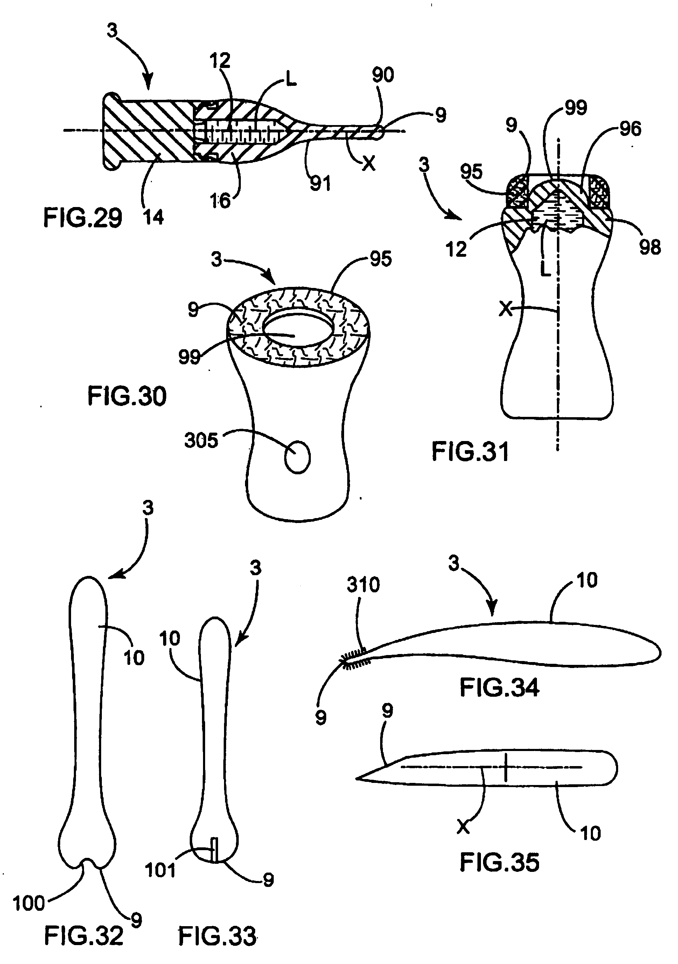

La surface d'application peut encore être définie par une tête 90 qui se raccorde au reste de l'applicateur par une portion étroite 91, comme illustré à la figure 29. Sur cette figure, la première partie 16, qui comporte la portion étroite 91, est par exemple réalisée dans un matériau métallique. La portion étroite 91 peut être flexible le cas échéant, et fléchir lors de l'utilisation.

La première partie 16 est par exemple encliquetée dans la deuxième partie 14, la cavité 12 étant formée à l'intérieur de la première partie 16 et obturée à une extrémité par la deuxième partie 14.

La surface d'application 9 de l'applicateur 3 peut être définie au moins partiellement par une pièce rapportée 95 réalisée par exemple dans un matériau élastiquement déformable, par exemple une mousse, comme représenté aux figures 30 et 31.

La pièce rapportée 95 peut présenter une forme annulaire et se monter sur une extension 96 du corps 98 de l'applicateur. Le sommet 99 de l'extension 96 peut également servir, le cas échéant, de surface d'application. La pièce 95 peut être nettoyée après avoir été utilisée.

On a illustré à la figure 30 la possibilité pour l'applicateur 3 de comporter un indicateur de température 305, lequel peut changer de couleur et signaler à l'utilisateur que la surface d'application 9 est à une température acceptable pour le traitement à effectuer.

La surface d'application 9 peut comporter, comme on le voit sur la figure 32, au moins un renfoncement 100 permettant une accumulation de produit, voire une fente 101 comme illustré à la figure 33.

La surface d'application 9 peut être définie à une extrémité par une pointe, comme illustré à la figure 34, voire par une face d'extrémité biseautée comme illustré à la figure 35.

On a également illustré à la figure 34 la possibilité pour la surface d'application 9 de comporter un flocage 310.

La surface d'application 9 peut encore être définie, comme illustré à la figure 36, par une masse 103 d'un matériau conducteur de la chaleur, fixée dans un logement 104 d'une première partie 105 de l'applicateur 3. Cette première partie peut définir la cavité 12 comportant le liquide L, et peut être supportée par une partie de préhension 106 réalisée dans un matériau moins bon conducteur de la chaleur que celui de la première partie 105. La masse 103 est par exemple une céramique, un verre, une pierre dense ou un matériau métallique.

On a illustré à la figure 37 la possibilité pour la surface d'application 9 d'être formée par une pièce 110 qui est en contact direct avec le liquide L contenu dans la cavité 12.

La pièce 110 est par exemple métallique et la cavité 12 est par exemple formée à l'intérieur d'un corps 111 en matière plastique isolante.

Un bouchon 112 peut être vissé sur le corps 111 pour fermer inférieurement la cavité 12.

L'applicateur 3 peut comporter deux surfaces d'application 9 ayant des formes différentes, comme illustré à la figure 38, par exemple une surface d'application ayant une forme arrondie et une surface d'application ayant une forme biseautée.

Une bague isolante 118 peut être prévue dans une région médiane de l'applicateur 3 pour définir la surface de préhension 10.

Les surfaces d'application 9 présentant des formes différentes sont par exemple situées à des extrémités opposées de l'applicateur 3.

Ce dernier peut encore comporter des surfaces d'application 9 ayant des formes différentes ou non, situées d'un même côté, comme illustré à la figure 39.

L'applicateur 3 peut par exemple présenter une forme de U, les extrémités du U définissant les surfaces d'application 9 et la base du U la surface de préhension 10.

L'applicateur 3 peut être agencé de manière à permettre, lorsqu'il repose sur une surface plane S, comme illustré à la figure 40, au liquide L contenu dans la cavité 12 de venir au contact de la paroi 120 qui définit la surface d'application 9.

On assure de cette manière un contact thermique entre le liquide L et la paroi 120, ce qui est avantageux notamment quand le liquide L change d'état et devient solide lorsque porté à une température suffisamment basse.

Au cours de l'utilisation, le liquide L à l'état solide peut rester au contact de la paroi 120 tant qu'il n'a pas fondu.

Le cas échéant, l'applicateur 3 peut être placé sur un support 130 lorsqu'il est mis au congélateur afin d'être maintenu tête en bas pendant la durée nécessaire à la congélation du liquide L, comme illustré à la figure 41.

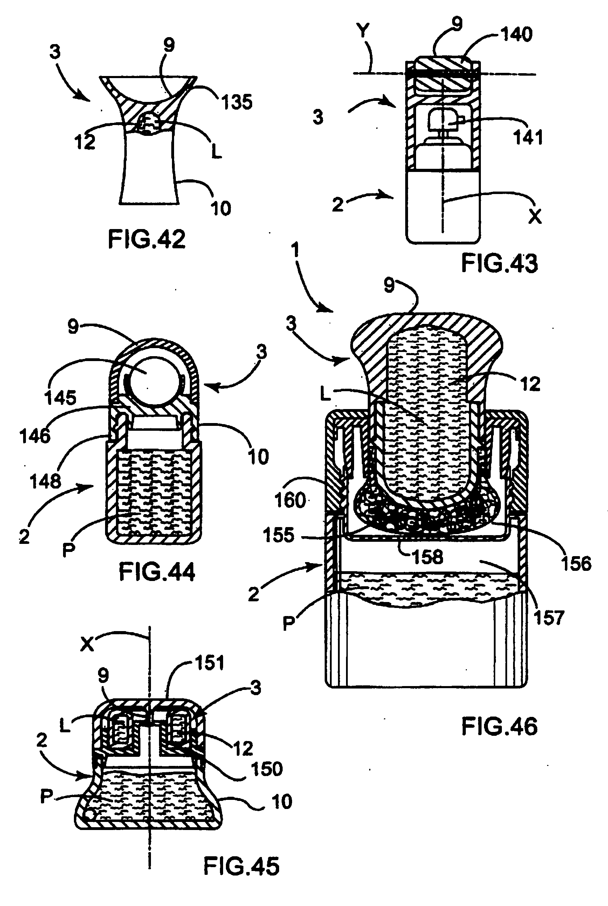

L'applicateur 3 peut être au moins partiellement élastiquement déformable et la surface d'application 9 peut être définie au moins partiellement par une lèvre flexible 135 permettant de créer un effet de ventouse, comme illustré à la figure 42.

La surface d'application 9 peut également être définie par un organe mobile, notamment rotatif, par exemple un rouleau 140 comme illustré à la figure 43, tournant autour d'un axe de rotation Y qui est par exemple perpendiculaire à l'axe longitudinal X.

On a illustré sur cette figure la possibilité pour le récipient 2 d'être équipé d'un dispositif de distribution 141 tel que par exemple une pompe ou une valve, permettant de distribuer la composition sur la région à traiter ou sur la surface d'application 9 préalablement à sa mise en contact avec la peau.

La surface d'application 9 peut encore être définie par un organe rotatif tel qu'une bille 145, comme illustré à la figure 44.

Cette bille peut être portée par un support 146 qui peut être fixé de manière amovible sur le récipient 2. Ainsi, l'utilisateur peut détacher le support 146 afin de le refroidir sans exposer au froid la composition P contenue dans le récipient. La bille peut, le cas échéant, comporter la cavité 12 et le liquide L.

Le support 146 peut être remis en place sur le récipient 2 une fois refroidi et le récipient 2 être utilisé comme organe de préhension.

En variante, c'est une jupe 148 de l'applicateur 3, qui permet sa fixation sur le récipient 2, qui peut définir la surface de préhension 10.

On a représenté à la figure 45 une variante de réalisation dans laquelle la surface d'application 9 peut être définie par un applicateur 3 de forme générale annulaire, pouvant être placé autour d'une cheminée 150 du récipient 2 par laquelle la composition P est distribuée.

Un organe de fermeture 151 peut être mis en place sur le récipient 2 de façon à obturer la cheminée 150 en l'absence d'utilisation. Pour utiliser l'applicateur 3, l'utilisateur enlève celui-ci du récipient 2 et le place par exemple au congélateur.

Ensuite, une fois refroidi, l'applicateur 3 est remis en place autour de la cheminée 150 et le récipient 2 peut être utilisé comme organe de préhension pour amener la surface d'application 9 au contact de la région à traiter.

Dans l'exemple de la figure 46, l'applicateur 3 comporte une surface d'application 9 destinée à apporter du froid dans la région à traiter et un organe 155 de prélèvement du produit contenu dans le récipient 2.

Cet organe de prélèvement 155 est par exemple situé sur l'applicateur 3 du côté opposé à la surface d'application 9.

Le récipient 2 comporte par exemple un logement 156 pour recevoir l'organe de prélèvement 155, ce logement 156 étant séparé d'un espace 157 contenant la composition P par une paroi ajourée 158 qui limite la quantité dont peut se charger l'organe de prélèvement 155. Ce dernier peut servir à appliquer la composition P sur la région à traiter, le cas échéant.

L'applicateur 3 peut être solidaire d'un organe de fermeture 160 du récipient 2, par exemple agencé pour se visser sur celui-ci.

Pour utiliser le dispositif 1 de la figure 46, l'utilisateur sépare l'applicateur 3 du récipient et place celui-ci au réfrigérateur ou congélateur. Ensuite, l'utilisateur prélève la composition P dans le logement 156, par exemple après avoir secoué le dispositif 1 avec l'applicateur 3 en place, et peut appliquer la composition contenue dans et/ou sur l'organe de prélèvement 155 au moyen de ce dernier. Une fois la composition appliquée, l'utilisateur peut retourner l'applicateur 3 et utiliser la surface d'application 9 pour apporter du froid.

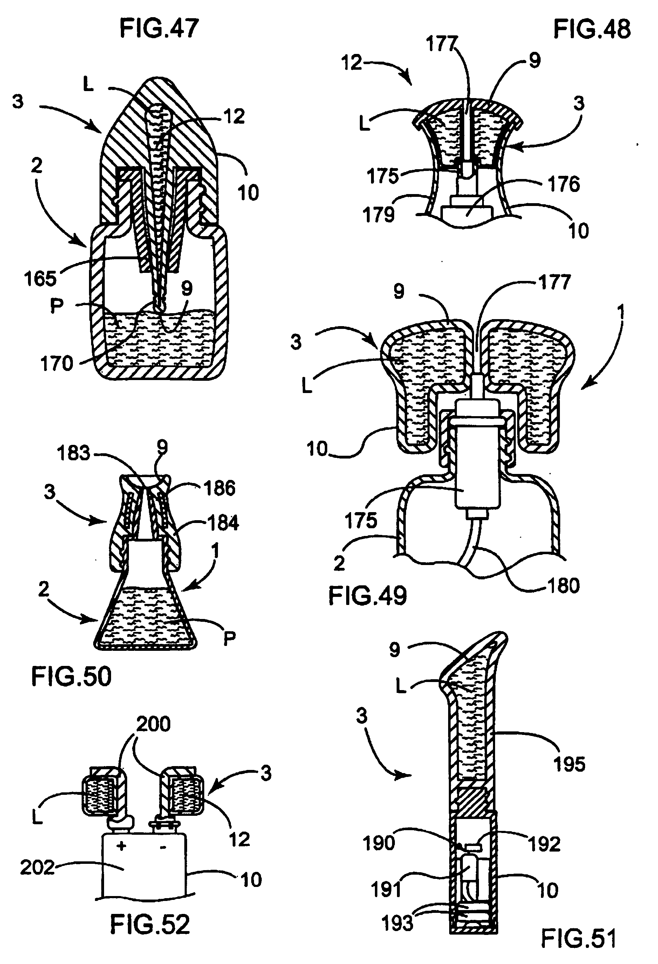

Dans l'exemple de la figure 47, la surface d'application 9 est agencée pour venir au contact de la composition du produit à l'intérieur du récipient 2. Ce dernier peut comporter un organe d'essorage 165 qui permet de retirer un excès de produit éventuellement présent sur l'applicateur 3.

L'applicateur 3 peut comporter des reliefs 170 permettant d'accroître la quantité de produit dont se charge l'applicateur et/ou permettant de conférer plus de souplesse à l'applicateur lors de son passage sur la région à traiter.

L'applicateur 3 peut être agencé pour se monter sur un dispositif de distribution 175 tel qu'une pompe ou valve, comportant une tige 176 dont l'enfoncement et/ou l'inclinaison provoque la distribution de la composition.

L'applicateur 3 peut comporter un canal intérieur 177 permettant au produit délivré par la tige 176 de gagner la surface d'application 9.

Dans l'exemple de la figure 48, l'applicateur 3 comporte une jupe d'habillage 179 permettant de dissimuler tout ou partie du dispositif de distribution 175 et définissant également une surface de préhension pour l'utilisateur.

Le dispositif 1 de la figure 48 s'utilise en séparant dans un premier temps l'applicateur 3 du dispositif de distribution 175 et en refroidissant l'applicateur 3 indépendamment du dispositif de distribution 175.

Ensuite, l'applicateur 3 est remis en place sur le dispositif de distribution 175 et l'utilisateur peut, en actionnant l'applicateur 3, provoquer la distribution de produit au travers du canal 177.

La figure 49 représente une variante dans laquelle le dispositif de distribution 175 comporte une pompe alimentée par un tube plongeur 180.

Dans l'exemple de la figure 50, l'applicateur 3 peut se visser sur le col d'un récipient et présente par exemple une surface d'application 9 convexe vers l'extérieur et dans le fond de laquelle débouche un orifice 183 d'amenée de la composition P. Le corps 184 de l'applicateur peut comporter un insert 186 destiné à accroître la capacité thermique de l'applicateur 3.

Quelle que soit la forme de l'applicateur 3, celui-ci peut être équipé d'un vibreur 190, comme illustré à la figure 51.

Un tel vibreur 190 comporte par exemple un moteur 191 entraînant en rotation une masse excentrée 192 de façon à provoquer des vibrations. Le moteur 191 peut être alimenté par une ou plusieurs piles 193, éventuellement rechargeables.

Le vibreur 190 est par exemple agencé pour se fixer de manière amovible sur une partie 195 de l'applicateur comportant la surface d'application 9, de façon à permettre à l'utilisateur d'utiliser le même vibreur avec des surfaces d'application 9 différentes.

L'applicateur 3 peut également comporter au moins une électrode, par exemple deux électrodes 200 afin de stimuler électriquement la région traitée lors de l'application. Ces électrodes sont par exemple reliées électriquement à une pile 202 qui définit la surface de préhension 10.

La cavité 12 s'étend par exemple autour des électrodes 200, lesquelles peuvent, le cas échéant, échanger de la chaleur avec le liquide L contenu dans cette cavité 12.

Pour utiliser l'applicateur 3 de la figure 52, l'utilisateur peut séparer l'applicateur 3 de la pile 202 et le placer au réfrigérateur.

Une fois la température souhaitée atteinte, l'utilisateur peut replacer l'applicateur 3 sur la pile 202 et se servir de cette dernière comme d'un organe de préhension pour amener les électrodes 200 au contact de la région à traiter. Cette dernière peut apporter du froid à la région traitée, le cas échéant.

Dans une variante, la pile 202 est refroidie également.

L'applicateur 3 peut également présenter des propriétés magnétiques.

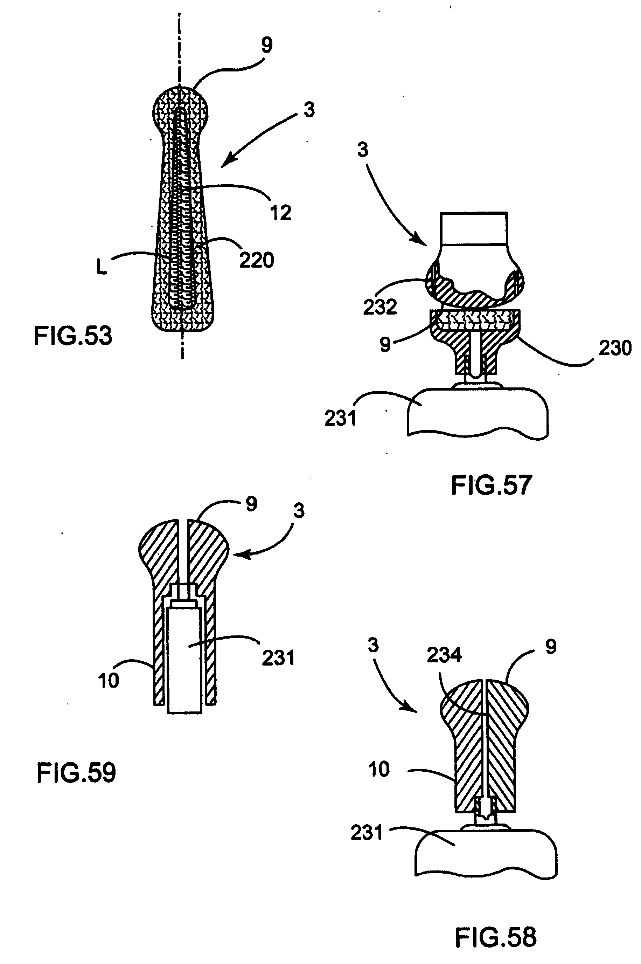

A titre d'exemple, on a représenté à la figure 53 un applicateur 3 qui comporte un corps 220 comportant une charge de particules magnétiques.

Ce corps 220 peut également définir comme dans l'exemple illustré au moins partiellement la cavité 12 contenant le liquide L. La présence d'une charge de particules magnétiques peut accroître, le cas échéant, la capacité thermique de l'applicateur 3.

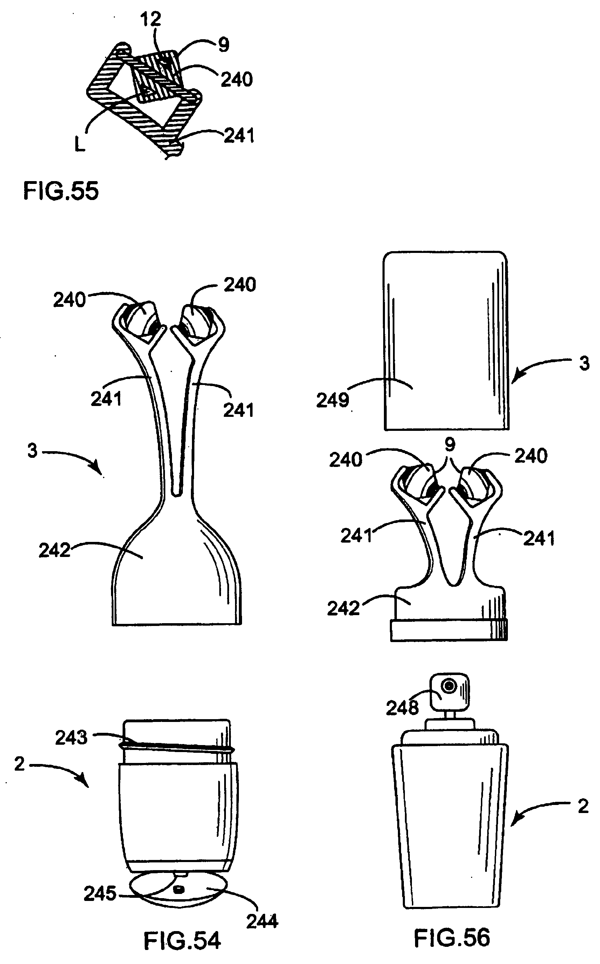

On a représenté aux figures 54, 55 et 63 un dispositif de conditionnement et d'application ayant un applicateur 3 comportant deux rouleaux 240 montés sur des tiges flexibles 241 reliées à un socle 242 permettant de rendre l'applicateur 3 solidaire du récipient 2.

Ce dernier comporte par exemple à une extrémité un filetage 243 pour la fixation de l'applicateur 3 sur le récipient 2 et à l'extrémité opposée un couvercle 244 permettant d'obturer un orifice de distribution 245.

Les rouleaux 240 sont agencés pour présenter la capacité thermique recherchée et peuvent présenter des cavités remplies du liquide L.

Sur la variante illustrée à la figure 63, les rouleaux sont constitués par deux parties réalisées en plastique qui s'emboîtent chacune de part et d'autre d'un disque 250 métallique. Deux cavités 12 contenant du liquide L sont ainsi formées de part et d'autre du disque. La tranche du disque définit une surface d'application 9. Les cavités sont délimitées dans leur majeure partie par une paroi en matière plastique.

Dans la variante illustrée à la figure 56, le récipient 2 est équipé d'un dispositif de distribution 248 tel qu'une pompe ou valve. La composition est par exemple contenue sous pression dans le récipient 2.

L'applicateur 3 peut comporter, comme illustré, un capot de protection 249 protégeant la surface d'application 9 en l'absence d'utilisation.

Dans les variantes illustrées aux figures 57 à 59, le refroidissement de l'applicateur 3 s'effectue par détente d'un gaz comprimé ou liquéfié.

Dans l'exemple de la figure 57, la surface d'application 9 est amenée au contact d'un bloc 230 poreux qui est exposé à une détente de gaz provenant d'un récipient aérosol 231. Le cas échéant, l'applicateur 3 peut comporter des canaux 232 permettant au gaz détendu de circuler à travers l'applicateur 3 et d'accroître ainsi la vitesse de refroidissement de celui-ci.

Dans l'exemple de la figure 58, la détente du gaz comprimé ou liquéfié s'effectue au travers de l'applicateur 3, celui-ci comportant par exemple un canal intérieur 234 pour la circulation du gaz froid détendu.

Le cas échéant, le récipient aérosol 231 peut être reçu au moins partiellement à l'intérieur de l'applicateur 3, comme illustré à la figure 59.

L'invention n'est pas limitée aux exemples qui viennent d'être décrits.

On peut notamment combiner entre elles les particularités de réalisation des divers exemples illustrés.

On peut par exemple équiper d'un vibreur l'un quelconque des applicateurs décrits.

L'expression « comportant un » doit être comprise comme étant synonyme de « comportant au moins un », sauf si le contraire est spécifié.

## Revendications

1. Ensemble comportant :
- une composition cosmétique ou dermatologique (P) contenue dans un récipient (2) ou substrat (52 ; 62), la composition étant destinée à être appliquée sur une région au moins du corps humain,
- un applicateur (3) à utiliser lors du traitement avec la composition cosmétique, l'applicateur ayant une surface d'application (9) définie au moins partiellement par un matériau présentant une inertie thermique d'au moins 1 000 Jm⁻² K⁻¹ S^{-½}, mieux d'au moins 5 000 Jm⁻² K⁻¹ S^{-½}, encore mieux d'au moins 10 000 Jm⁻² K⁻¹ S^{-½}, et préférentiellement d'au moins 20 000 Jm⁻² K⁻¹ S^{-½}, et/ou par un matériau présentant une conductivité thermique supérieure ou égale à 1 Wm⁻¹ K⁻¹, mieux 40 Wm⁻¹ K⁻¹, encore mieux 180 Wm⁻¹ K⁻¹,
l'applicateur et le récipient ou substrat étant contenus dans un dispositif de conditionnement commun, **caractérisé en ce que** l'applicateur comporte une cavité (12) contenant un composé liquide (L).

2. Ensemble selon la revendication 1, l'applicateur (3) comportant un matériau de densité volumique supérieure ou égale à 1,5 g/cm³ définissant au moins partiellement la surface d'application (9).

3. Ensemble selon la revendication 2, le matériau étant un métal, une pierre ou du verre.

4. Ensemble selon l'une quelconque des revendications précédentes, l'applicateur comportant un matériau de capacité calorifique massique supérieure ou égale à 500 J kg⁻¹ K⁻¹, mieux à 1 000 J kg⁻¹ K⁻¹, encore mieux à 2 000 J kg⁻¹ K⁻¹.

5. Ensemble selon l'une quelconque des revendications précédentes, l'applicateur présentant une masse supérieure ou égale à 15 g.

6. Ensemble selon l'une quelconque des revendications précédentes, l'applicateur présentant une surface de préhension (10) définie au moins partiellement par un matériau présentant une conductivité thermique inférieure ou égale à 1 Wm⁻¹K⁻¹, mieux inférieure ou égale à 0,5 Wm⁻¹K⁻¹, encore mieux à 0,1 Wm⁻¹ K⁻¹.

7. Ensemble selon l'une quelconque des revendications précédentes, la surface d'application ayant au moins une zone d'étendue comprise entre 3 et 10 cm² pouvant venir entièrement au contact avec la peau et la capacité thermique de l'applicateur étant supérieure ou égale à 250 J K⁻¹ à une température de 15 °C.

8. Ensemble selon l'une quelconque des revendications précédentes, la surface d'application ayant au moins un zone d'étendue comprise entre 0,1 et 3 cm² pouvant venir entièrement au contact avec la peau et la capacité thermique de l'applicateur étant supérieure ou égale à 50 J K⁻¹ à une température de 15 °C.

9. Ensemble selon l'une quelconque des revendications précédentes, l'applicateur (3) étant agencé pour se fixer de manière amovible sur le récipient (2).

10. Ensemble selon la revendication 9, l'applicateur étant agencé pour se fixer de manière amovible sur un organe de fermeture (6) du récipient.

11. Ensemble selon l'une quelconque des revendications 1 à 9, le substrat (62) étant agencé pour se fixer de manière amovible sur l'applicateur (3).

12. Ensemble selon l'une quelconque des revendications précédentes, l'applicateur comportant un organe d'application rotatif agencé pour venir au contact de la région à traiter.

13. Ensemble selon l'une quelconque des revendications précédentes, l'applicateur étant au moins partiellement magnétique.

14. Ensemble selon l'une quelconque des revendications précédentes, l'applicateur comportant une lèvre flexible destinée à venir au contact de la région à traiter.

15. Ensemble selon la revendication 9, l'applicateur comportant un passage permettant à la composition d'être distribuée quand l'applicateur est monté sur le récipient.

16. Ensemble selon l'une quelconque des revendications précédentes, l'applicateur comportant un vibreur.

17. Ensemble selon l'une quelconque des revendications précédentes, l'applicateur comportant une électrode reliée à une source électrique.

18. Ensemble selon l'une quelconque des revendications 1 à 15, l'applicateur étant dépourvu de source électrique ou de moyen de raccordement à une source électrique.

19. Ensemble selon l'une quelconque des revendications précédentes, la surface d'application étant définie au moins partiellement par une pièce amovible (95), notamment une pièce au moins partiellement en un matériau absorbant.

20. Ensemble selon la revendication 1, le composé liquide pouvant changer d'état lorsque refroidi de la température ambiante à une température non inférieure à -18 °C.

21. Ensemble selon la revendication 1 ou 20, le composé liquide comportant de l'eau, voire étant constitué par de l'eau.

22. Ensemble selon l'une des revendications 1 ou 20 à 21, la cavité (12) comportant au moins 0,2 cm³ de liquide, mieux entre 1 et 80 cm³ de liquide.

23. Ensemble selon l'une des revendications 1 ou 20 à 22, la cavité étant délimitée en partie par un matériau présentant une conductivité thermique supérieure ou égale à 1 Wm⁻¹ K⁻¹ et en partie par un matériau présentant une conductivité thermique inférieure ou égale à 1 Wm⁻¹ K⁻¹.

24. Ensemble selon l'une quelconque des revendications 1 ou 20 à 23, la cavité étant délimitée en partie par un matériau métallique et en partie par un matériau plastique.

25. Ensemble selon la revendication 24, la plus grande partie de la cavité étant délimitée par un matériau présentant une conductivité thermique inférieure ou égale à 1 Wm⁻¹ K⁻¹.

26. Ensemble selon l'une quelconque des revendications 1 ou 20 à 25, l'applicateur comportant entre la cavité (12) et la surface d'application (9) une paroi ayant une plus petite épaisseur (eₘᵢₙ) inférieure ou égale à 50 mm, notamment comprise entre 0,1 mm et 50 mm, mieux inférieure ou égale à 1 mm, encore mieux à 0,5 mm.

27. Ensemble selon l'une quelconque des revendications 1 ou 20 à 26, étant agencé pour, en position de stockage, pouvoir reposer sur une surface de manière à ce que le liquide soit en contact avec la paroi délimitant la surface d'application.

28. Ensemble selon l'une quelconque des revendications précédentes, étant agencé pour, lorsque refroidi à -8 °C et appliqué sur la peau, conserver pendant au moins 30 s, mieux 1 mn, encore mieux 15 mn ou 30 mn, une température inférieure ou égale à 15 °C.

29. Ensemble selon l'une quelconque des revendications précédentes, l'applicateur (3) ne comportant pas de composés réagissant ensemble selon une réaction endothermique.

30. Ensemble selon l'une quelconque des revendications précédentes, la surface d'application (9) étant au moins partiellement recouverte d'une membrane élastomère, d'une mousse, d'un flocage, d'un film de matière plastique, d'une éponge, d'un feutre, d'un tissé ou non-tissé.

31. Ensemble selon l'une quelconque des revendications précédentes, la cavité étant délimitée par une paroi d'épaisseur non constante.

32. Applicateur selon l'une quelconque des revendications 1 ou 31, les surfaces d'application et de préhension étant définies respectivement par deux parties (14, 16) assemblées l'une sur l'autre.

33. Applicateur selon l'une quelconque des revendications 1 ou 31 à 32, la cavité étant délimitée en partie par un matériau métallique et en partie par un matériau plastique.

34. Applicateur selon la revendication 1, la plus grande partie de la cavité étant délimitée par un matériau présentant une conductivité thermique inférieure ou égale à 1 Wm⁻¹ K⁻¹.

35. Applicateur selon l'une quelconque des revendications 1 ou 31 à 34, comportant entre la cavité (12) et la surface d'application (9) une paroi ayant une plus petite épaisseur (eₘᵢₙ) inférieure ou égale à 50 mm, notamment comprise entre 0,1 mm et 50 mm, mieux inférieure ou égale à 1 mm, encore mieux à 0,5 mm.

36. Applicateur selon l'une quelconque des revendications 1 ou 31 à 35, configuré pour pouvoir reposer de manière à ce que le liquide soit en contact avec la paroi délimitant la surface d'application.

37. Applicateur selon l'une quelconque des revendications 1 ou 31 à 36, la surface d'application (9) étant au moins partiellement recouverte d'une membrane élastomère, d'une mousse, d'un flocage, d'un film de matière plastique, d'une éponge, d'un feutre, d'un tissé ou non-tissé.

38. Applicateur selon l'une quelconque des revendications 1 ou 31 à 37, la surface interne de la paroi délimitant la cavité étant recouverte d'un vernis.

39. Procédé de traitement cosmétique, non thérapeutique, d'au moins une région du corps humain, comportant les étapes suivantes :
a) refroidir à une basse température inférieure ou égale à 15 °C un applicateur (3) tel que défini dans l'une quelconque des revendications précédentes, puis
b) charger l'applicateur ainsi refroidi avec une composition cosmétique (P) à une température plus proche de la température ambiante que celle de l'applicateur et ayant avec cette dernière une différence d'au moins 5 °C, et appliquer la composition en utilisant l'applicateur, ou
c) avant ou après l'étape a), appliquer sur la région à traiter au moins une composition cosmétique et après l'étape a) amener l'applicateur (3) ainsi refroidi au contact de la région à traiter.

40. Procédé selon la revendication 39, comportant l'application sur la région à traiter d'un substrat (52) portant la composition, l'applicateur étant amené au contact du substrat ainsi appliqué.

41. Procédé selon la revendication 40, l'applicateur étant refroidi en étant introduit dans un réfrigérateur ou congélateur.

## Claims

1. Assembly comprising:
- a cosmetic or dermatological composition (P) contained in a recipient (2) or substrate (52; 62), the composition being intended to be applied to at least one region of the human body;
- an applicator (3) to be used during the treatment with the cosmetic composition, the applicator having an application surface (9) defined at least partially by a material having a thermal inertia of at least 1000 J m⁻² K⁻¹ s^{-1/2}, better of at least 5000 J m⁻² K⁻¹ s^{-1/2}, better still of at least 10 000 J m⁻² K⁻¹ s^{-1/2}, and preferably of at least 20 000 J m⁻² K⁻¹ s^{-1/2}, and/or by a material having a thermal conductivity greater than or equal to 1 W m⁻¹ K⁻¹, better 40 W m⁻¹ K⁻¹, better still 180 W m⁻¹ K⁻¹,
the applicator and the recipient or substrate being contained in a common packaging device, **characterized in that** the applicator has a cavity (12) containing a liquid compound (L).

2. Assembly according to Claim 1, the applicator (3) comprising a material having a density per unit volume greater than or equal to 1.5 g/cm³ at least partially defining the application surface (9).

3. Assembly according to Claim 2, the material being a metal, a stone or glass.

4. Assembly according to any one of the preceding claims, the applicator comprising a material having a heat capacity per unit mass greater than or equal to 500 J kg⁻¹ K⁻¹, better 1000 J kg⁻¹ K⁻¹, better still 2000 J kg⁻¹ K⁻¹.

5. Assembly according to any one of the preceding claims, the applicator having a mass greater than or equal to 15 g.

6. Assembly according to any one of the preceding claims, the applicator having a gripping surface (10) defined at least partially by a material having a thermal conductivity less than or equal to 1 W m⁻¹ K⁻¹, better less than or equal to 0.5 W m⁻¹ K⁻¹, better still 0.1 W m⁻¹ K⁻¹.

7. Assembly according to any one of the preceding claims, the application surface having at least one zone with an area of between 3 and 10 cm² which may come completely in contact with the skin and the heat capacity of the applicator being greater than or equal to 250 J K⁻¹ at a temperature of 15°C.

8. Assembly according to any one of the preceding claims, the application surface having at least one zone with an area of between 0.1 and 3 cm² which may come completely in contact with the skin and the heat capacity of the applicator being greater than or equal to 50 J K⁻¹ at a temperature of 15°C.

9. Assembly according to any one of the preceding claims, the applicator (3) being arranged in order to be attached in a removable manner to the recipient (2).

10. Assembly according to Claim 9, the applicator being arranged in order to be attached in a removable manner to a closure member (6) of the recipient.

11. Assembly according to any one of Claims 1 to 9, the substrate (62) being arranged in order to be attached in a removable manner to the applicator (3).

12. Assembly according to any one of the preceding claims, the applicator comprising a rotary application member arranged in order to come into contact with the region to be treated.

13. Assembly according to any one of the preceding claims, the applicator being at least partially magnetic.

14. Assembly according to any one of the preceding claims, the applicator comprising a flexible lip intended to come in contact with the region to be treated.

15. Assembly according to Claim 9, the applicator comprising a passage that enables the composition to be distributed when the applicator is mounted on the recipient.

16. Assembly according to any one of the preceding claims, the applicator comprising a vibrator.

17. Assembly according to any one of the preceding claims, the applicator comprising an electrode connected to a power supply.

18. Assembly according to any one of Claims 1 to 15, the applicator being without a power supply or a means of connection to a power supply.

19. Assembly according to any one of the preceding claims, the application surface being at least partially defined by a removable component (95), especially a component made at least partially of an absorbent material.

20. Assembly according to Claim 1, the liquid compound possibly changing state when cooled from ambient temperature to a temperature not below -18°C.

21. Assembly according to Claim 1 or 20, the liquid compound comprising water or even being constituted by water.

22. Assembly according to one of Claims 1 or 20 to 21, the cavity (12) comprising at least 0.2 cm³ of liquid, better between 1 and 80 cm³ of liquid.

23. Assembly according to one of Claims 1 or 20 to 22, the cavity being delimited in part by a material having a thermal conductivity greater than or equal to 1 W m⁻¹ K⁻¹ and in part by a material having a thermal conductivity less than or equal to 1 W m⁻¹ K⁻¹.

24. Assembly according to any one of Claims 1 or 20 to 23, the cavity being delimited in part by a metallic material and in part by a plastic material.

25. Assembly according to Claim 24, the largest part of the cavity being delimited by a material having a thermal conductivity less than or equal to 1 W m⁻¹ K⁻¹.

26. Assembly according to any one of Claims 1 or 20 to 25, the applicator comprising, between the cavity (12) and the application surface (9), a wall having a smaller thickness (eₘᵢₙ) less than or equal to 50 mm, especially between 0.1 mm and 50 mm, better less than or equal to 1 mm, better still 0.5 mm.

27. Assembly according to any one of Claims 1 or 20 to 26, being arranged in order, in the storage position, to be able to rest on a surface in such a way that the liquid is in contact with the wall delimiting the application surface.

28. Assembly according to any one of the preceding claims, being arranged in order, when cooled to -8°C and applied to the skin, to retain for at least 30 s, better 1 min, better still 15 min or 30 min, a temperature less than or equal to 15°C.

29. Assembly according to any one of the preceding claims, the applicator (3) not comprising compounds that react together according to an endothermic reaction.

30. Assembly according to any one of the preceding claims, the application surface (9) being at least partially covered by an elastomer membrane, a foam, a flocking, a plastic film, a sponge, a felt, a woven fabric or a non-woven fabric.

31. Assembly according to any one of the preceding claims, the cavity being delimited by a wall of non-constant thickness.

32. Applicator according to any one of Claims 1 to 31, the application and gripping surfaces being defined respectively by two parts (14, 16) joined to one another.

33. Applicator according to any one of Claims 1 or 31 to 32, the cavity being delimited in part by a metallic material and in part by a plastic material.

34. Applicator according to Claim 1, the largest part of the cavity being delimited by a material having a thermal conductivity less than or equal to 1 W m⁻¹ K⁻¹.

35. Applicator according to any one of Claims 1 or 31 to 34, comprising, between the cavity (12) and the application surface (9), a wall having a smaller thickness (eₘᵢₙ) less than or equal to 50 mm, especially between 0.1 mm and 50 mm, better less than or equal to 1 mm, better still 0.5 mm.

36. Applicator according to any one of Claims 1 or 31 to 35, configured in order to be able to rest in such a way that the liquid is in contact with the wall delimiting the application surface.

37. Applicator according to any one of Claims 1 or 31 to 36, the application surface (9) being at least partially covered by an elastomer membrane, a foam, a flocking, a plastic film, a sponge, a felt, a woven fabric or a non-woven fabric.

38. Applicator according to any one of Claims 1 or 31 to 37, the inner surface of the wall delimiting the cavity being covered with a varnish.

39. Process for the cosmetic, non-therapeutic treatment of at least one region of the human body, comprising the following steps:
a) cooling an applicator (3) as defined in any one of the preceding claims, to a low temperature less than or equal to 15°C; then
b) loading the applicator thus cooled with a cosmetic composition (P) at a temperature closer to ambient temperature than that of the applicator and having with the latter temperature a difference of at least 5°C, and applying the composition by using the applicator; or
c) before or after step a), applying at least one cosmetic composition to the region to be treated and after step a) bringing the applicator (3) thus cooled into contact with the region to be treated.

40. Process according to Claim 39, comprising the application to the region to be treated of a substrate (52) bearing the composition, the applicator being brought into contact with the substrate thus applied.

41. Process according to Claim 40, the applicator being cooled by being introduced into a refrigerator or freezer.

## Patentansprüche

1. Einheit, die umfasst:
- eine kosmetische oder dermatologische Zusammensetzung (P), die in einem Behälter (2) oder Substrat (52; 62) enthalten ist, wobei die Zusammensetzung dafür vorgesehen ist, auf mindestens einen Bereich des menschlichen Körpers aufgetragen zu werden,
- einen Applikator (3), der bei der Behandlung mit der kosmetischen Zusammensetzung verwendet wird, wobei der Applikator eine Anwendungsoberfläche (9) hat, die mindestens teilweise durch ein Material, das eine Wärmeträgheit von mindestens 1000 J m⁻² K⁻¹ s^{-1/2}, besser mindestens 5000 J m⁻² K⁻¹ s^{-1/2}, noch besser mindestens 10000 J m⁻² K⁻¹ s^{-1/2} und vorzugsweise mindestens 20000 J m⁻² K⁻¹ s^{-1/2} aufweist, und/oder durch ein Material definiert ist, das eine Wärmeleitfähigkeit von 1 W m⁻¹ K⁻¹ oder darüber, besser 40 W m⁻¹ K⁻¹ oder darüber und noch besser 180 W m⁻¹ K⁻¹ oder darüber aufweist,
wobei der Applikator und der Behälter oder das Substrat in einer üblichen Verpackungsvorrichtung enthalten sind, **dadurch gekennzeichnet, dass** der Applikator einen Hohlraum (12) umfasst, der eine flüssige Verbindung (L) enthält.

2. Einheit nach Anspruch 1, wobei der Applikator (3) ein Material mit einer Dichte von 1,5 g/cm³ oder darüber umfasst, das mindestens teilweise die Anwendungsoberfläche (9) definiert.

3. Einheit nach Anspruch 2, wobei das Material ein Metall, ein Stein oder aus Glas ist.

4. Einheit nach einem der vorhergehenden Ansprüche, wobei der Applikator ein Material mit einer spezifischen Wärmekapazität von 500 J kg⁻¹ K⁻¹ oder darüber, besser von 1000 J kg⁻¹ K⁻¹ oder darüber und noch besser von 2000 J kg⁻¹ K⁻¹ oder darüber umfasst.

5. Einheit nach einem der vorhergehenden Ansprüche, wobei der Applikator eine Masse von 15 g oder darüber aufweist.

6. Einheit nach einem der vorhergehenden Ansprüche, wobei der Applikator eine Greifoberfläche (10) aufweist, die mindestens teilweise durch ein Material definiert ist, das eine Wärmeleitfähigkeit, von 1 W m⁻¹ K⁻¹ oder darunter, besser von 0,5 W m⁻¹ K⁻¹ oder darunter, noch besser von 0,1 W m⁻¹ K⁻¹ oder darunter aufweist.

7. Einheit nach einem der vorhergehenden Ansprüche, wobei die Anwendungsoberfläche mindestens eine Zone mit einer Ausdehnung aufweist, die im Bereich von 3 bis 10 cm² liegt, die vollständig mit der Haut in Kontakt treten kann, wobei die Wärmekapazität des Applikators bei einer Temperatur von 15 °C größer als oder gleich 250 J K⁻¹ ist.

8. Einheit nach einem der vorhergehenden Ansprüche, wobei die Anwendungsoberfläche mindestens eine Zone mit einer Ausdehnung aufweist, die im Bereich von 0,1 bis 3 cm² liegt, die vollständig mit der Haut in Kontakt treten kann, wobei die Wärmekapazität des Applikators bei einer Temperatur von 15 °C größer als oder gleich 50 J K⁻¹ ist.

9. Einheit nach einem der vorhergehenden Ansprüche, wobei der Applikator (3) so angeordnet wird, dass er abnehmbar auf dem Behälters (2) befestigt ist.

10. Einheit nach Anspruch 9, wobei der Applikator so angeordnet wird, dass er abnehmbar auf einem Schließorgan (6) des Behälters befestigt ist.

11. Einheit nach einem der Ansprüche 1 bis 9, wobei das Substrat (62) so zusammengefügt wird, dass es abnehmbar auf dem Applikator (3) fixiert wird.

12. Einheit nach einem der vorhergehenden Ansprüche, wobei der Applikator ein drehbares Anwendungsorgan umfasst, das so angeordnet wird, dass es mit dem zu behandelnden Bereich in Kontakt kommt.

13. Einheit nach einem der vorhergehenden Ansprüche, wobei der Applikator mindestens teilweise magnetisch ist.

14. Einheit nach einem der vorhergehenden Ansprüche, wobei der Applikator eine flexible Lippe umfasst, die dafür vorgesehen ist, mit dem zu behandelnden Bereich in Kontakt zu treten.

15. Einheit nach Anspruch 9, wobei der Applikator einen Durchlass aufweist, der es der Zusammensetzung ermöglicht, verteilt zu werden, wenn der Applikator auf dem Behälter montiert ist.

16. Einheit nach einem der vorhergehenden Ansprüche, wobei der Applikator einen Vibrator umfasst.

17. Einheit nach einem der vorhergehenden Ansprüche, wobei der Applikator eine Elektrode umfasst, die mit einer Stromquelle verbunden ist.

18. Einheit nach einem der Ansprüche 1 bis 15, wobei der Applikator keine Stromquelle oder Einrichtung zu Verbindung mit einer Stromquelle aufweist.

19. Einheit nach einem der vorhergehenden Ansprüche, wobei die Anwendungsoberfläche mindestens teilweise durch ein abnehmbares Teil (95) definiert ist, insbesondere ein Teil, das mindestens teilweise aus einem absorbierenden Material besteht.

20. Einheit nach Anspruch 1, wobei die flüssige Verbindung ihren Zustand ändern kann, wenn sie von Umgebungstemperatur auf eine Temperatur nicht unterhalb von -18 °C gekühlt wird.

21. Einheit nach Anspruch 1 oder 20, wobei die flüssige Verbindung Wasser umfasst oder sogar aus Wasser besteht.

22. Einheit nach einem der Ansprüche 1 oder 20 bis 21, wobei der Hohlraum (12) mindestens 0,2 cm³ Flüssigkeit und besser 1 bis 80 cm³ Flüssigkeit umfasst.

23. Einheit nach einem der Ansprüche 1 oder 20 bis 22, wobei der Hohlraum teilweise durch ein Material begrenzt wird, das eine Wärmeleitfähigkeit von 1 W m⁻¹ K⁻¹ oder darüber aufweist, und teilweise durch ein Material begrenzt wird, das eine Wärmeleitfähigkeit von 1 W m⁻¹ K⁻¹ oder darunter aufweist.

24. Einheit nach einem der Ansprüche 1 oder 20 bis 23, wobei der Hohlraum teilweise durch ein metallisches Material und teilweise durch ein Kunststoffmaterial begrenzt wird.

25. Einheit nach Anspruch 24, wobei der größte Teil des Hohlraums durch ein Material begrenzt wird, das eine Wärmeleitfähigkeit von 1 W m⁻¹ K⁻¹ oder darunter aufweist.

26. Einheit nach einem der Ansprüche 1 oder 20 bis 25, wobei der Applikator zwischen dem Hohlraum (12) und der Anwendungsoberfläche (9) eine Wand aufweist, die eine kleinste Dicke (eₘᵢₙ) von 50 mm oder darunter, insbesondere im Bereich von 0,1 mm bis 50 mm, besser von 1 mm oder darunter, noch besser von 0,5 mm aufweist.

27. Einheit nach einem der Ansprüche 1 oder 20 bis 26, die so zusammengefügt ist, dass sie in der Lagerungsposition so auf einer Oberfläche ruhen kann, dass sich die Flüssigkeit im Kontakt mit der Wand befindet, die die Anwendungsoberfläche begrenzt.

28. Einheit nach einem der vorhergehenden Ansprüche, die so zusammengefügt ist, dass sie, wenn sie auf -8 °C abgekühlt ist und auf die Haut aufgebracht wird, mindestens 30 s, besser 1 min, noch besser 15 min oder 30 min, eine Temperatur von 15 °C oder darunter behält.

29. Einheit nach einem der vorhergehenden Ansprüche, wobei der Applikator (3) keine Verbindungen umfasst, die miteinander in einer endothermen Reaktion reagieren.

30. Einheit nach einem der vorhergehenden Ansprüche, wobei die Anwendungsoberfläche (9) mindestens teilweise von einer Elastomermembran, einem Schaumstoff, einer Beflockung, einer Folie aus einem Kunststoffmaterial, einem Schwamm, einem Filz, einem Gewebe oder einem Vlies bedeckt ist.

31. Einheit nach einem der vorhergehenden Ansprüche, wobei der Hohlraum von einer Wand mit einer nicht konstanten Dicke begrenzt wird.

32. Applikator nach einem der Ansprüche 1 oder 31, wobei die Anwendungsoberfläche und die Greifoberfläche jeweils durch zwei Teile (14, 16) definiert werden, die aufeinander montiert sind.

33. Applikator nach einem der Ansprüche 1 oder 31 bis 32, wobei der Hohlraum teilweise durch ein metallisches Material und teilweise durch ein Kunststoffmaterial begrenzt wird.

34. Applikator nach Anspruch 1, wobei der größte Teil des Hohlraums von einem Material begrenzt wird, das eine Wärmeleitfähigkeit von 1 W m⁻¹ K⁻¹ oder darunter aufweist.

35. Applikator nach einem der Ansprüche 1 oder 31 bis 34, der zwischen dem Hohlraum (12) und der Anwendungsoberfläche (9) eine Wand aufweist, die eine kleinste Dicke (eₘᵢₙ) von 50 mm oder darunter, insbesondere im Bereich von 0,1 mm bis 50 mm, besser von 1 mm oder darunter, noch besser von 0,5 mm aufweist.

36. Applikator nach einem der Ansprüche 1 oder 31 bis 35, der so konfiguriert ist, dass er so ruhen kann, dass sich die Flüssigkeit im Kontakt mit der Wand befindet, die die Anwendungsoberfläche begrenzt.

37. Applikator nach einem der Ansprüche 1 oder 31 bis 36, wobei die Anwendungsoberfläche (9) mindestens teilweise von einer Elastomermembran, einem Schaumstoff, einer Beflockung, einer Folie aus einem Kunststoffmaterial, einem Schwamm, einem Filz, einem Gewebe oder einem Vlies bedeckt ist.

38. Applikator nach einem der Ansprüche 1 oder 31 bis 37 wobei die innere Oberfläche der Wand, die den Hohlraum begrenzt, mit einem Lack überzogen ist.

39. Verfahren zur nicht therapeutischen kosmetischen Behandlung mindestens eines Bereichs des menschlichen Körpers, das die folgenden Schritte umfasst:
a) Abkühlen eines Applikators (3), der wie in einem der vorhergehenden Ansprüche definiert ist, auf eine Temperatur von 15 °C oder darunter, dann
b) Befüllen des so abgekühlten Applikators mit einer kosmetischen Zusammensetzung (P) mit einer Temperatur, die näher bei Umgebungstemperatur liegt als die Temperatur des Applikators und die zu dieser Temperatur einen Unterschied von mindestens 5 °C aufweist, und Auftragen der Zusammensetzung unter Anwendung des Applikators, oder
c) vor oder nach Schritt a) Auftragen mindestens einer kosmetischen Zusammensetzung auf die zu behandelnde Region und nach Schritt a) Inkontaktbringen des so abgekühlten Applikators (3) mit dem zu behandelnden Bereich.

40. Verfahren nach Anspruch 39, die das Auftragen eines Substrats (52), dass die Zusammensetzung trägt, auf den zu behandelnden Bereich, wobei der Applikator mit dem so aufgetragenen Substrat in Kontakt gebracht wird.

41. Verfahren nach Anspruch 40, wobei der Applikator abgekühlt wird, indem er in einen Kühlschrank oder einen Gefrierschrank eingebracht wird.
